# EUROPÄISCHE PATENTSCHRIFT

(11) **EP 1 715 806 B1**
(45) Veröffentlichungstag und Bekanntmachung des Hinweises auf die Patenterteilung: **27.01.2016**
(21) Anmeldenummer: 05700338.6
(22) Anmeldetag: 28.01.2005
(51) Int. Cl.: A61C 8/00, A61F 2/30, A61B 17/68

(54) **IN KNOCHENGEWEBE ZU IMPLANTIERENDES IMPLANTAT UND VERFAHREN ZU DESSEN HERSTELLUNG**
IMPLANT THAT CAN BE IMPLANTED IN OSSEOUS TISSUE AND METHOD FOR PRODUCING THE SAME
IMPLANT A IMPLANTER DANS UN TISSU OSSEUX ET SON PROCEDE DE FABRICATION

(30) Priorität: 20.02.2004 CH 287042004
(43) Veröffentlichungstag der Anmeldung: 02.11.2006
(73) Patentinhaber: Woodwelding AG, 6362 Stansstad (CH)
(72) Erfinder: MAYER, Jörg, 5702 Niederlenz (CH); AESCHLIMANN, Marcel, 2514 Ligerz (CH); TORRIANI, Laurent, 2516 Lamboing (CH); RAST, Christopher, 2505 Biel (CH); MÜLLER, Andrea, 8413 Neftenbach (CH)
(74) Vertreter: Frei Patent Attorneys
(86) Internationale Anmeldenummer: PCT/CH2005/000043
(87) Internationale Veröffentlichungsnummer: WO 2005/079696

(56) Entgegenhaltungen:
- EP-A- 0 806 192
- WO-A-02/069817
- DE-A1- 19 735 103
- US-A- 4 547 157

## Beschreibung

Die Erfindung liegt im Bereiche der Medizinaltechnik und betrifft ein in Knochengewebe zu implantierendes Implantat, das ein standardisiertes Implantat sein kann, das in eine eigens dafür erstellte oder dafür angepasste Kavität implantiert wird, oder ein Individualimplantat, das in eine individuelle Knochenkavität implantiert wird (z.B. Dentalimplantat, Gelenkimplantat, oder ein Implantat zur Füllung eines Knochendefektes). Die Erfindung betrifft ferner Verfahren zur Herstellung und zur Implantation des Implantats.

In Knochengewebe zu implantierende Implantate werden üblicherweise in Knochenkavitäten implantiert, wobei eine solche Knochenkavität eine für die Implantation eigens hergestellte Kavität (z.B. Bohrung oder Stufenbohrung) oder eine durch andere Umstände, wie beispielsweise Trauma oder degenerative Krankheit entstandene Kavität ist. Dabei wird das Implantat gemäss dem Stande der Technik entweder mit Hilfe von um das Implantat angeordnetem Zement in die Kavität eingepasst oder es weist eine entsprechend genau an die Kavität angepasste Form auf, derart, dass es nach der Implantation über einen möglichst grossen, funktionell wesentlichen Anteil seiner Oberfläche mit dem Knochengewebe in direktem Kontakt steht. Im Falle eines Individualimplantats bedeutet das Letztere, dass die Implantatform eine unregelmässige ist, insbesondere ein unregelmässiger Konus, der nicht durchgängig einen runden Querschnitt und/oder keine gerade Achse hat.

Dentalimplantate, die als Ersatz einer natürlichen Zahnwurzel zum Tragen von zum Beispiel künstlichen Kronen, Abutments, Brücken oder Zahnprothesen nach Extraktion der natürlichen Zahnwurzel im Kieferknochen implantiert werden, sind sowohl als genormte, in eigens dafür erstellte oder mindestens angepasste Kavitäten zu implantierende Implantate als auch als an die Form einer individuellen Zahnwurzel oder Alveole angepasste Individualimplantate bekannt.

Genormte, in eigens dafür erstellte Bohrungen zu implantierende Dentalimplantate sind zylindrische oder leicht konische, im wesentlichen rotationssymmetrische Stifte, meist Schrauben, die in verschiedenen Grössen und Formen auf den Markt kommen und von denen der Dentalchirurg das für einen spezifischen Fall am besten geeignete Implantat auswählt. Die Implantation eines solchen Dentalimplantates ist im allgemeinen erst dann möglich, wenn sich die Kavität, die durch Extraktion der zu ersetzenden, natürlichen Zahnwurzel entstanden ist, mit regeneriertem Knochengewebe gefüllt hat, also erst um eine erste Wartezeit von 3 bis 6 Monaten nach der genannten Extraktion. Das eingeschraubte Implantat wird unmittelbar nach der Implantation üblicherweise nicht belastet, denn das Risiko ist gross, dass durch die Belastung das Implantat relativ zum Knochengewebe zu stark bewegt wird, derart, dass durch diese Bewegungen eine gute Integration des Implantates im Knochengewebe (Osseointegration) verhindert wird. Aus diesem Grunde wird in einer grossen Mehrheit von Fällen ein aus dem Kiefer ragender Teil (Krone, Brücke etc.) erst nach einer zweiten Wartezeit von wiederum ca. 3 bis 6 Monaten auf dem Implantat aufgebaut, nämlich erst dann, wenn das Implantat voll im Knochengewebe integriert ist und durch normale Belastung bedingte Relativbewegungen zwischen Implantat und Knochengewebe ein physiologisch verträgliches Mass nicht mehr überschreiten.

Es zeigt sich, dass schraubenförmige Dentalimplantate, wenn sie voll im Kieferknochen integriert sind, eine Stabilität aufweisen, die für normale Belastungen genügt und die auch langfristig unverändert bleibt. Dies ist unter anderem auch darauf zurückzuführen, dass das Implantat durch das Gewinde seitlich im Knochengewebe gut verankert ist, wodurch Schubkräfte auf das Knochengewebe reduziert werden und eine Überbelastung des Alveolengrundes durch Druckkräfte vermieden wird.

Es ist bekannt, dass Knochengewebe die Tendenz hat, sich während den Wartezeiten, in denen das Dentalimplantat bzw. der Kieferknochen örtlich nicht belastet ist, in unerwünschter Weise zurück zu bilden. Es ist auch bekannt, dass Relativbewegungen zwischen Implantat und Knochengewebe, die ein physiologisch verträgliches Mass nicht überschreiten, die Knochenregeneration und damit die Osseointegration des Implantates anregen würden. Aus diesem Grunde wird mit verschiedensten Mitteln versucht, die genannten Wartezeiten zu verkürzen oder ganz zu umgehen.

Um die erste Wartezeit, nämlich die Zeit, die es dauert, bis eine durch die Extraktion der natürlichen Zahnwurzel entstandene Kavität sich mit Knochengewebe gefüllt hat, zu vermeiden und auch um die die natürliche Kavität (Alveole) umgebende, verdichtete Knochenschicht (alveolärer Knochen) als tragendes Element ausnützen zu können, wird vorgeschlagen, dem Implantat eine Form zu geben, die nicht wie eine Schraube im wesentlichen rotationssymmetrisch ist sondern im wesentlichen der Form der zu ersetzenden, natürlichen Zahnwurzel entspricht (Indi vidualimplantat). Ein solches Implantat kann unmittelbar oder kurz nach der Extraktion der natürlichen Zahnwurzel in die bestehende Kavität (natürliche Alveole) implantiert werden.

Da aber im natürlichen Zustand zwischen der Zahnwurzel und der Alveolenwand ein faseriger Halteapparat (Zahnhaut) angeordnet ist, hat ein Implantat, das ein genaues Abbild der natürlichen Zahnwurzel darstellt (z.B. durch Negativ-Positiv-Giessverfahren hergestellt), in der Alveole keinen festen Sitz. Dies wirkt sich negativ aus auf die Osseointegration während der zweiten Wartezeit, da sich in einem Spalt zwischen Alveolenwand und Implantat bindegewebeartiges Gewebe bilden kann, das die Osseointegration mindestens örtlich verhindert, dem Implantat aber keine genügende Stabilität geben kann.

Um mit solchen, einer natürlichen Zahnwurzel nachgebildeten Dentalimplantaten eine verbesserte Stabilität für die Osseointegrations-Phase (zweite Wartezeit) und damit eine bessere Ausgangslage für eine erfolgreiche Osseointegration zu geben, wird gemäss US-5562450 (Gieloff et al.) und WO-88/03391 (Lundgren) das Implantat gegenüber der natürlichen Zahnwurzel überdimensioniert, das heisst, mit etwas grösseren Querschnitten ausgebildet, und die mit dem Knochen in Kontakt kommende Implantatoberfläche wird mit Strukturen, insbesondere mit Vertiefungen (Wabenstrukturen, Strukturen mit Hinterschnitten) versehen. Die genannten Implantate werden beispielsweise hergestellt, indem nach der Extraktion der natürlichen Zahnwurzel diese oder die Alveole beispielsweise berührungslos vermessen wird, indem die Messdaten in einem CAD-System bearbeitet werden und indem in einem CAM-System aus einem entsprechenden Rohling anhand der bearbeiteten Messdaten durch Fräsen, Schleifen, Elektroerosion etc. das Implantat erstellt wird.

Die genannten, 'überdimensionierten' individuellen Dentalimplantate sitzen nach der Implantation durch 'press fit' bedeutend fester in der Alveole als genaue Abbilder der natürlichen Zahnwurzel. Es zeigt sich aber, dass die Alveolenwand innerhalb kurzer Zeit durch Umbauvorgänge und mechanische Relaxation gegenüber den eingebrachten Kräften relaxiert und dass damit das Implantat nicht mehr durch ,pressfit' stabilisiert ist sondern wiederum relativ locker in der Alveole sitzt, so dass, trotzt verbesserter Primärstabilität unmittelbar nach der Implantation, keine optimalen Bedingungen für eine Osseointegration gegeben sind. Es zeigt sich ebenfalls, dass diese Implantate auch nach der Osseointegrations-Phase (zweite Wartezeit) dazu tendieren, bei Belastung ihren Halt im Kieferknochen wieder zu verlieren. Wie an der 52. Jahrestagung der Deutschen Gesellschaft für Zahnärzliche Prothetik und Werkstoffkunde (DGZPW) im Mai 2003 von R.-J. Kohal et al. vorgetragen wurde (veröffentlicht in DentSci (2) 7: 11), bildet sich der Kieferknochen im Bereich solcher Implantate während der Osseointegrationsphase und bei nachfolgender Belastung stark zurück und können sich die Implantate auch ganz aus dem Knochengewebe lösen.

Die oben genannten Befunde können nicht nur für Dentalimplantate sondern allgemein für in Knochenkavitäten zu implantierende Implantate unter anderem darauf zurückgeführt werden, dass das Implantat grossflächig im Kontakt mit einer sich durch einen Eingriff (Zahnextraktion) in intensivem Umbau befindlichen Knochengewebe befindet, so dass die im Knochen induzierten Spannungen sehr gering sind. Die Oberflächengeometrien können über den Pressfit die Spannungen zwar sehr lokal erhöhen, aber das beanspruchte Volumen ist offenbar zu klein um effektiv eine mechanisch induzierte Stimulation der Knochenneubildung zu erreichen. Die durch die Belastung (Kaubewegungen) entstehenden Druckkräfte auf das Implantat bewirken in der Kavitätswand vor allem Schubkräfte. Durch den Formfit mit der Kavitätswand wird zudem kaum eine Stabilisierung gegenüber Rotationskräften erreicht. Als Folge davon kann es in der Grenzfläche zum sich neu bildenden Knochen zu Verschiebungen kommen, welche eine Osseointegration mangels ausreichender Rotationsstabilität verhindert. Diese Problematik ist vor allem auch in der Hüftendoprothetik eingehend diskutiert worden. Für den speziellen Fall des Zahnimplantates ist aufgrund der Steilheit der Alveolenwand ein Transfer der axialen Belastung nur in geringem Masse möglich. Damit verschieben sich die Belastungen vom ursprünglich proximalen Teil der Alveole stark nach distal und können in der Folge zu Überbelastung des Alveolengrundes führen, welcher ja bei der Extraktion als Austrittstelle für die Blutgefässe und Nerven nicht vollständig verknöchert ist. Folgen können Drucknekrosen und weitere durch Fehlbelastungen induzierte Probleme sein. Im Design herkömmlicher Schraubenimplantate wird aber dieser Problematik grösste Beachtung geschenkt, obschon in diesem Fall die Alveole normalerweise vollständig verknöchert ist.

Dokument WO 02/069817 offenbart ein Knochenimplantat, das für die Implantation in einer Implantationsrichtung parallel zu einer Implantatsachse in einer von einer Kavitätswand aus Knochengewebe umgebenen Kavität geeignet ist, wobei das Implantat einen, zu implantierenden Bereich aufweist, wobei der zu implantierende Bereich ferner kantenartige Vorsprünge aufweist, die nicht in einer mit der Implantatsachse gemeinsamen Ebene liegen und die sich mindestens teilweise um den Umfang des Implantats erstrecken, wobei das Implantat in einem zu implantierenden Bereich Oberflächenbereiche aufweist, die durch Auspressen eines verflüssigbaren Materials aus einem Hohlraum mit offenen Poren erstellbar sind.

Zusammenfassend kann festgestellt werden, dass von den bekannten, zementlos anwendbaren Knochenimplantaten die schraubenförmigen Implantate gegenüber allen anders geformten Implantaten bezüglich Stabilität vorzuziehen sind, dass sie aber durch die unausweichbaren, geometrischen Bedingungen, die bei ihrer Anwendung zu erfüllen sind, vielfach nicht anwendbar oder nur unter Inkaufnahme anderer Nachteile anwendbar sind. Ähnliches gilt auch für viele andere, in Knochengewebe zu implantierende Implantate.

Die Aufgabe der Erfindung besteht nun darin, ein in Knochengewebe zu implantierendes Implantat (Individualimplantat oder genormtes Implantat) zu schaffen sowie Verfahren zu dessen Herstellung und Implantation. Das erfindungsgemässe Implantat soll in eingeheiltem Zustand eine mindestes so gute Stabilität aufweisen wie ein in eine entsprechende Bohrung eingeschraubtes, schraubenförmiges Implantat, die Primärstabilität (unmittelbar nach der Implantation) des erfindungsgemässen Implantates soll insbesondere gegen rotative Belastungen aber bedeutend besser sein als für das genannte schraubenförmige Implantat. Insbesondere soll aber das erfindungsgemässe Implantat bezüglich geometrischer Bedingungen bedeutend weniger limitiert sein als das genannte schraubenförmige Implantat. Dabei soll die Implantation des erfindungsgemässen Implantates mit an sich bekannten Implantationsmethoden durchführbar sein und soll das Implantat in an sich bekannten Herstellungsschritten herstellbar sein.

Diese Aufgaben werden gelöst durch das Implantat und die Verfahren, wie sie in den entsprechenden Patentansprüchen definiert sind.

Das erfindungsgemässe Implantat ist in Anspruch 1 definiert und wird im wesentlichen parallel zu einer Implantatsachse (also ohne wesentliche Rotation) implantiert und hat einen in Implantationsrichtung vorlaufenden, distalen Endbereich und einen auf der Implantatsachse dem distalen Endbereich gegenüberliegenden, proximalen Endbereich, der nach der Implantation im Bereich der Knochenoberfläche positioniert ist oder gegebenenfalls aus dem Knochen ragt. Des Implantat weist an seinen zwischen dem distalen und dem proximalen Endbereich liegenden Oberflächenbereichen, die durch die Implantation mindestens teilweise mit dem Knochengewebe in Kontakt gebracht werden (zu implantierender Bereich), spanbildende Schneidekanten auf, die nicht mit der Implantatsachse in einer gemeinsamen Ebene verlaufen, also bei der Implantation nicht in Richtung ihrer Länge sondern im wesentlichen quer dazu im Knochengewebe bewegt werden, und die gegen den distalen Endbereich gerichtet sind. Erfindungsgemäss sind die Schneidekanten als Aussenkanten stufenartiger Reduktionen des Querschnitts zum distalen Endbereich hin ausgebildet, so dass sich das Implantat in distaler Richtung verjüngt, oder der zu implantierende Bereich ist im Wesentlichen zylindrisch und die Schneidekanten ragen aus der Zylinderform und weisen in Implantationsrichtung abnehmende Abstände von der Implantatsachse auf. Zusätzlich weist das Implantat ein durch mechanische Vibration verflüssigbares, beispielsweise thermoplastisches Material auf, das in Oberflächenbereichen ohne Schneidekanten angeordnet ist oder das in einem Hohlraum des Implantates positioniert oder positionierbar ist, wobei der Hohlraum durch Öffnungen mit Oberflächenbereichen ohne Schneidekanten verbunden ist.

Das erfindungsgemässe Implantat wird in der Richtung der Implantatsachse ohne wesentliche Rotation in die Knochenkavität eingebracht, wobei die Schneidekanten die Knochenoberfläche spanbildend anschneiden. Gleichzeitig mit der Einbringung des Implantates in die Knochenkavität wird das Implantat mit mechanischen Vibrationen beaufschlagt. Dadurch wird das verflüssigbare Material, das in diesem Falle vorzugsweise ein thermoplastisches Material ist, an Berührungsstellen mit dem Knochenmaterial verflüssigt und in Unebenheiten und Poren oder auch in spezifisch für diesen Zwecke erstellte Strukturen der Kavitätswand eingepresst, wobei es in einen innigen Kontakt mit der Knochenoberfläche gebracht wird. Nach einer Wiederverfestigung des verflüssigbaren Materials bildet dieses formschlüssige und gegebenenfalls stoffschlüssige Verbindungen zwischen dem Implantat und dem Knochengewebe.

Wenn das verflüssigbare Material in einem Hohlraum des Implantates positioniert ist, wird die mechanische Vibration vorteilhafterweise erst nach der Einbringung des Implantates in die Kavität angewendet und zwar nur auf das verflüssigbare Material. Für ein derartig ausgestaltetes Implantat kann als verflüssigbares Material ebenfalls ein thermoplastisches Material zur Anwendung kommen oder aber ein thixotroper, partikulärer, hydraulischer oder polymerer Zement, wie er in der Orthopädie auch für die Verankerung von Implantaten oder zum Beispiel auch für die Infiltration von osteoporotischen, kollabierten Wirbelkörpern verwendet wird.

Das erfindungsgemässe Implantat ist unmittelbar nach der Implantation in der Kavität stabilisiert durch seine Verbindung mit dem Knochengewebe mittels verflüssigbarem Material, wobei diese Stabilisierung gegen Druck- und Zugbelastungen (also etwa parallel zur Implantatsachse) wirksam ist aber auch gegen rotativ wirkende Belastungen. Auch die sich bei der Implantation in das Knochengewebe einschneidenden Schneidekanten verankern das Implantat. Sowohl die Verankerung durch das verflüssigbare Material als auch durch die Schneidekanten wirken insbesondere auf die Seitenwände der Kavität, sodass der Kavitätsgrund wenig oder nicht belastet ist, was insbesondere für Dentalimplantate wichtig ist. Alle genannten Effekte geben dem erfindungsgemässen Implantat eine Primärstabilität, die in den meisten Fällen für eine Belastung unmittelbar nach der Implantation genügt. Die Verbindungsstrukturen aus dem thermoplastischen Material haben ein geringeres Elastizitätsmodul als die Knochenmatrix und insbesondere als das Implantat selbst und sie sind kriechfähig, was sie besonders vorteilhaft macht für schockartige Belastungen und zum Abbau von übermässigen Spannungen. Durch ihre Elastizität erlauben diese Verbindungen kleine und dadurch osseointegrations-fördernde Relativbewegungen zwischen Implantat und Knochengewebe die insbesondere im Bereich der Schneidekanten stimulierend auf das Knochengewebe wirken. Gleichzeitig verhindern diese Verbindungen aber grössere Verschiebungen zwischen Implantat und Knochengewebe, welche zur Störung des Osseointegrationsprozesses führen würden.

Da das erfindungsgemässe Implantat im wesentlichen ohne Rotation (insbesondere ohne Rotation, die mehr als 360° beträgt) implantiert wird, ist es möglich und vorteilhaft, das Implantat derart zu formen, dass es auch durch seine Form gegen rotativ wirkende Belastungen in der Kavität stabilisiert ist. Wie noch zu zeigen sein wird, ist es aber trotzdem möglich, ein erfindungsgemässes Implantat derart auszugestalten, dass es in einer Kavität mit rundem Querschnitt (Bohrung oder Stufenbohrung) implantierbar ist.

Wenn das erfindungsgemässe Implantat ein Individualimplantat ist, wird es in den meisten Fällen die Form eines unregelmässigen (nicht rotationssymmetrischen) Konus aufweisen, das heisst, sich gegen sein distales Ende hin verjüngen und für den Fall eines Dentalimplantates eine im wesentlichen an eine natürliche Zahnwurzel angepasste Form haben. Ein solches individuelles Dentalimplantat gemäss Erfindung ist wie bekannte, einer natürlichen Zahnwurzel nachgebildete Dentalimplantate unmittelbar nach der Extraktion der natürlichen Zahnwurzel implantierbar, ist aber im Gegensatz zu bekannten, auch als Zahnreplika bezeichneten, individuellen Dentalimplantaten während der Osseointegrations-Phase und nach dieser langfristig stabil, wie dies für schraubenförmige Dentalimplantate der Fall ist. Dasselbe gilt auch für individuelle Gelenkimplantate und Implantate für die Reparatur von individuellen Knochendefekten gemäss Erfindung.

Die Schneidekanten eines sich gegen das distale Ende hin verjüngenden Implantates gemäss Erfindung, z.B. eines individuellen Dentalimplantates sind als Aussenkanten stufenartiger Querschnittsreduktionen ausgebildet und sind auch in diesem Falle relativ zur Kavität derart zu dimensionieren, dass sie nach der Implantation mindestens teilweise in der Kavitätswand eingeschnitten sind.

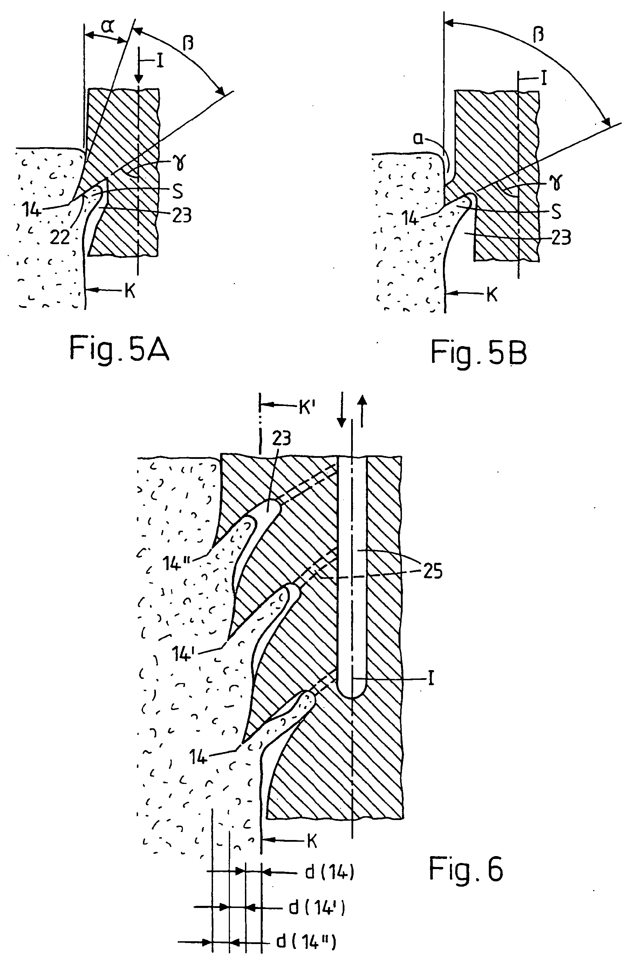

Die Schneidekanten oder die als Schneidekanten ausgerüsteten, stufenartigen Querschnittsreduktionen erstrecken sich ganz oder teilweise um das Implantat herum, im wesentlichen senkrecht oder schief zur Implantatsachse und weisen einen Keilwinkel auf, der weniger gross ist als 90° (siehe Fig. 5). Zusätzlich zu den Schneidekanten kann ein konisch ausgebildetes Implantat auch stufenartige Querschnittsreduktionen ohne schneidende Wirkung (Keilwinkel 90° oder grösser) aufweisen.

Wenn die genannten, stufenförmigen Querschnittsreduktionen keine Schneidekanten aufweisen und/oder relativ tief sind, ist es vorteilhaft, in der Kavität vor der Implantation entsprechende Schultern beispielsweise mit Hilfe eines formmässig auf das Implantat abgestimmten Werkzeuges zu erstellen. Die zu wählende Vorgehensweise mit oder ohne Vorerstellung von Schultern in der Kavität ist insbesondere abhängig von der Beschaffenheit des vorliegenden Knochengewebes aber auch von Chirurg und Patient. Mit vorgängigem Erstellen der Schultern wird (bei gleicher Tiefe der Querschnittsreduktionen) das Knochengewebe bei der Implantation mechanisch weniger belastet, so dass sich diese Vorgehensweise insbesondere eignet für die Anwendung bei älteren Patienten mit schlechter Knochenqualität.

Im implantierten Zustand bilden die in das Knochengewebe der Kavitätswandung eingeschnittenen Schneidekanten des erfindungsgemässen Implantates, ähnlich wie die Gewindegänge eines schraubenförmigen Implantates, seitliche Abstützungen im Knochengewebe, also Stellen, an denen Druckkräfte seitlich vom Implantat in das Knochengewebe eingekoppelt werden und zwar mehr orthogonal als dies über eine konische im wesentlichen glatte Implantatsoberfläche ohne Schneidekanten und Stufen der Fall sein kann und insbesondere mehr als dies bei einer zylindrischen Implantatsoberfläche der Fall ist. Durch diese Abstützungen entstehen gezielte, lokale Belastungsstellen, an denen Knochenregeneration stimuliert wird.

Zusätzlich zu den oben beschriebenen Strukturen kann das erfindungsgemässe Implantat auch in einer gemeinsamen Ebene mit der Implantatsachse, also im wesentlichen in Implantationsrichtung verlaufende, furchende oder selbstschneidende Strukturen aufweisen, die bei der Implantation in die Kavitätswand eindringen und dem Implantat insbesondere bezüglich Torsionskräften Primärstabilität verleihen. In ähnlicher Weise kann auch ein erfindungsgemässes Implantat im proximalen Bereiche einen schneidenden Kragen aufweisen, durch den das Implantat zusätzlich in der Oberfläche des kortikalen Knochens stabilisiert wird.

Die Oberflächenbereiche der Schneidekanten an einem erfindungsgemässen Implantat bestehen aus einem für eine schneidende Wirkung auf Knochenmaterial geeigneten Material, das sich unter den Implantationsbedingungen nicht verflüssigt. Sie bestehen beispielsweise aus Titan, aus einer Titanlegierung, aus Zirkonoxid oder aus einem anderen geeigneten metallischen oder keramischen Werkstoff oder aus einem entsprechend verstärkten Kunststoff.

Das im erfindungsgemässen Implantat anzuwendende, verflüssigbare Material ist vorteilhafterweise biologisch resorbierbar. Es erstreckt sich nicht über die Oberflächenbereiche mit den Schneidekanten, welche Oberflächenbereiche biokompatible, das heisst knochenfreundliche und vorteilhafterweise osseointegrative Eigenschaften haben. In diesen Oberflächenbereichen kann die Osseointegration des Implantates unmittelbar nach der Implantation beginnen und die Verankerung mittels resorbierbarem thermoplastischem Material sukzessive ablösen. Es ist aber auch möglich, ein nicht resorbierbares, thermoplastisches Material zu verwenden, derart, dass dessen Verankerung im Knochengewebe die Verankerung mittels Osseointegration dauernd ergänzen oder gar ersetzen kann. In diesem Fall kann auch eine weitergehende Bedeckung der Oberfläche mit dem Polymer zur Maximierung der Stabilität sinnvoll sein.

Für das erfindungsgemässe Individualimplantat geeignete, biologisch resorbierbare, verflüssigbare Materialen sind thermoplastische Polymere auf Milch- und/oder Glukolsäurebasis (PLA, PLLA, PGA, PLGA etc) oder Polyhydroxyalkanoate (PHA), Polycaprolactone (PCL), Polysaccharide, Polydioxanone (PD), Polyanhydride, Polypeptide, Trimethylcarbonat (TMC) oder entsprechende Copolymere oder Mischpolymere oder die genannten Polymere enthaltende Verbundwerkstoffe. Geeignete, nicht resorbiebare, thermoplastische Materialen sind beispielsweise: Polyolefine (z.B. Polyethylen), Polyacrylate, Polymetacrylate, Polycarbonate, Polyamide, Polyester, Polyurethane, Polysulfone, Polyphenylsufide oder Flüssig-Kristall-Polymere (liqid cristal polymers oder LCPs), Polyacetale, halogenierte Polymere, insbesondere halogenierte Polyolefine, Polyphenylsulfide, Polysulfone, Polyether oder entsprechende Copolymere und Mischpolymere oder die genannten Polymere enthaltende Verbundwerkstoffe.

Insbesondere geeignet sind als resorbierbare, verflüssigbare Materialien: Poly-LDL-Lactid (z.B. erhältlich von Böhringer unter dem Handelsnamen Resomer LR708) oder Poly-DL-Lactid (z.B. erhältlich von Böhringer unter den Handelsnamen Resomer R208); als nicht resorbierbares, verflüssigbares Material: Polyamid 11 oder Polyamid 12.

Die wichtigsten Vorteile des erfindungsgemässen Implantates sind die folgenden:
- Da das erfindungsgemässe Implantat im wesentlichen ohne Rotation um die Implantatsachse implantierbar ist, kann es formmässig an eine existierende Kavität angepasst werden, beispielsweise an eine Alveole, in die es im wesentlichen unmittelbar nach der Extraktion der natürlichen Zahnwurzel implantiert werden kann. Das heisst für den Patienten, dass eine Wartezeit zwischen Extraktion und Implantation entfällt. Ferner entfallen aufwendige Massnahmen zur genauen Ausrichtung des Dentalimplantates und weiterer, auf dem Implantat aufzubauender Teile (Abutment, Krone etc.)
- Im Falle eines Dentalimplantates, das formmässig an eine natürliche Zahnwurzel angepasst ist, bleibt bei der Implantation die Alveolenwand als Bereich mit einer verdichteten Knochenstruktur grossteils erhalten und kann das Implantat besser abstützen als dies für von der Alveole weiter entferntes, weniger dichtes Knochengewebe der Fall ist.
- Da das Implantat dank seiner Verankerung durch das verflüssigbare Material und durch den Eingriff der Schneidekanten in das Knochenmaterial und dank seiner Form, die eine Rotation in der Kavität verhindert, unmittelbar nach der Implantation für eine Belastung genügend stabilisiert ist, kann es unmittelbar nach der Implantation belastet werden.
- Da ein erfindungsgemässes Dentalimplantat im wesentlichen unmittelbar nach der Implantation belastbar ist, kann es als im wesentlichen einstückiges Implantat einen ganzen Zahn mit Wurzelbereich und Kronenbereich darstellen. Es entfallen weitere im Mund des Patienten durchzuführende Arbeitsschritte für den Aufbau des Implantates.
- Durch die seitliche Abstützung des Implantates über die Schneidekanten in der Kavitätswand werden Druckkräfte auf das Implantat lokal gut in das Knochengewebe eingekoppelt, wodurch das Implantat eine LangzeitStabilität erhält, die der Langzeitstabilität eines schraubenförmigen Implantates gleichkommt.
- Da die seitliche Abstützung des Implantates im Knochengewebe der Kavitätswand eine Abstützung auf dem Kavitätsgrund verhindert oder mindestens relevant reduziert, werden Komplikationen am Kavitätsgrund vermieden, was vor allem für Dentalimplantate wichtig ist, da der Alveolengrund nicht für grosse Belastungen ausgerüstet ist.
- Durch die Belastung des Implantates unmittelbar nach der Implantation wird durch Nicht-Belastung bedingte Knochenrückbildung vermieden.
- Durch die Verankerung des Implantates mit Hilfe des verflüssigbaren Materials werden belastungsbedingte Relativbewegungen zwischen Implantat und Knochengewebe auf ein physiologisches Mass reduziert und dadurch die Osseointegration nicht nur nicht gestört sondern gefördert.
- Durch Verwendung eines nicht abbaubaren, verflüssigbaren Materials wird eine starke Langzeit-Verankerung des Implantates auch in durch Krankheit oder altersbedingten Abbau schwachem oder wenig regenerationsfähigem Knochengewebe möglich.

Verschiedene, beispielhafte Ausführungsformen des erfindungsgemässen Implantates sowie dessen Herstellung und Implantation werden im Zusammenhang mit den folgenden Figuren im Detail beschrieben. Dabei zeigen:
- **Figur 1**: einen natürlichen Zahn im Schnitt quer zum Kieferkamm;
- **Figur 2**: ein erfindungsgemässes, individuelles Dentalimplantat, das den Zahn gemäss Figur 1 ersetzt;
- **Figur 3**: eine Seitenansicht einer bevorzugten Ausführungsform des individuellen Dentalimplantates gemäss Erfindung;
- **Figur 4**: drei aufeinander projizierte Querschnitte durch ein individuelles Dentalimplantat (etwa Schnittlinien A-A, B-B und C-C in Fig. 3);
- **Figuren 5A und 5B**: axiale Schnitte durch den Bereich einer Schneidekante eines erfindungsgemässen Implantates:
- **Figur 6**: ein axialer Schnitt durch eine Serie von in Implantationsrichtung hintereinander angeordneten Schneidekanten eines erfindungsgemässen Implantats;
- **Figur 7**: ein axialer Schnitt durch eine stufenförmige Querschnittsreduktion ohne Schneidekante eines erfindungsgemässen Implantates;
- **Figur 8**: ein axialer Schnitt durch eine stufenförmige Querschnittsreduktion mit darüber verlaufendem, verflüssigbarem Material;
- **Figur 9**: ein axialer Teilschnitt durch eine Ausführungsformen des erfindungsgemässen Implantats mit in einem inneren Hohlraum positioniertem, verflüssigbarem Material;
- **Figuren 10 bis 12**: drei beispielhafte Dentalimplantate gemäss Erfindung;
- **Figuren 13 und 14**: ein erfindungsgemässes Implantat, das für die Implantation in eine Bohrung geeignet ist als Seitenansicht (Fig. 13) und quer zur Implantatsachse geschnitten (Fig. 14, Schnittlinie XIV-XVI in Fig. 13);
- **Figuren 15, 16A und 16B**: ein weiteres, erfindungsgemässes Implantat, das für die Implantation in eine Bohrung geeignet ist und in dem das verflüssigbare Material in einem inneren Hohlraum positioniert ist, axial geschnitten (Fig. 15), quer zur Implantatsachse geschnitten (Fig. 16A, Schnittlinie XVI-XVI in Fig. 15) und als Seitenansicht (Fig. 16B);
- **Figuren 17 und 18**: Details von Implantaten gemäss Figuren 15 und 16.
- **Figur 19**: ein Implantat gemäss Figuren 13 und 14 mit einem Übergangsstück;
- **Figur 20**: eine beispielhafte Ausführungsform für eine Spielpassung als Verbindung zwischen Implantat und Übergangsstück oder zwischen Übergangsstück und Sonotrode (axialer Schnitt);
- **Figur 21**: ein Schema zur Illustration der Herstellung eines individuellen Dentalimplantates gemäss Erfindung;
- **Figur 22**: ein Schema zur Illustration der Implantation eines Dentalimplantates gemäss Erfindung;
- **Figur 23A bis 23C**: eine schematische Illustration der Implantation eines Gelenkimplantates gemäss Erfindung;
- **Figuren 24A bis 24C**: eine schematische Illustration der Reparatur einer durch einen Knochentumor erzeugten Defektstelle mit Hilfe eines erfindungsgemässen Implantates.

In allen Figuren sind dieselben Elemente mit denselben Bezugsnummern bezeichnet.

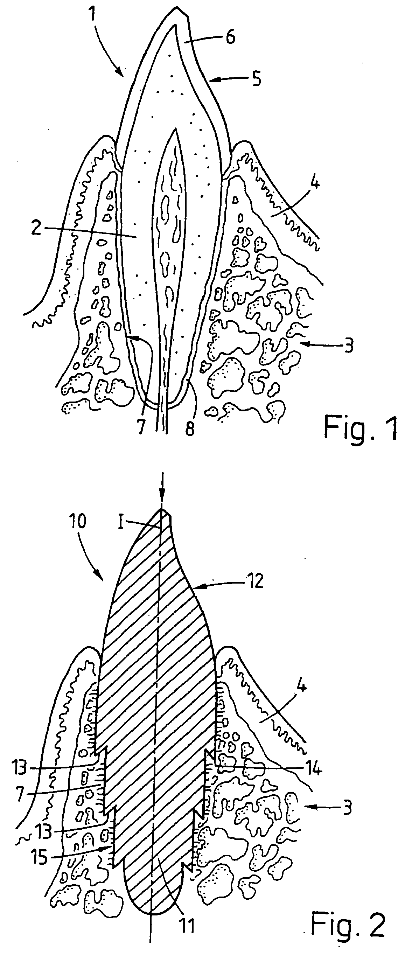

**Figur 1** zeigt in einem Schnitt quer zum Kieferkamm einen natürlichen Zahn 1, dessen Wurzel 2 in einem Kieferknochen 3 eingewachsen ist. Der Kieferknochen 3 ist von Zahnfleisch 4 (Bindegewebe und Haut) bedeckt. Die Zahnkrone 5 ragt über den Kieferknochen und über das Zahnfleisch 4 hinaus und ist mit einer Schmelzschicht 6 bedeckt, während das Innere der Zahnkrone 5 und die Zahnwurzel 2 aus Dentin (Zahnbein) bestehen. Zur Aufnahme der Zahnwurzel 2 weist der Kieferknochen 3 eine Alveole (Zahnfach) auf, wobei das Knochengewebe der Alveolenwand 7 (alveolärer Knochen) verglichen mit Knochengewebe, das weiter von der Wurzel 2 entfernt ist, üblicherweise eine höhere Dichte und dadurch eine höhere mechanische Stabilität aufweist. Zwischen der Alveolenwand 7 und der Zahnwurzel 2 liegt die Zahnhaut 8, die Collagenfasern (Scharpäischer Faserapparat) enthält, mit deren Hilfe die Zahnwurzel 2 mit der Alveolenwand 7 verbunden ist, die also den Zahn tragen und auf den Zahn wirkende Kräfte seitlich in das Knochengewebe einkoppeln. Bei einer Extraktion des Zahnes wird die Zahnhaut zerstört. Sie ist nicht regenerierbar.

**Figur 2** zeigt in einem gleichen Schnitt wie Figur 1 ein individuelles Dentalimplantat 10 gemäss Erfindung, das den in der Figur 1 dargestellten Zahn 1 ersetzt, das also an der Stelle dieses Zahnes 1 im Kieferknochen 3 implantiert ist (Implantationsrichtung bzw. Implantatsachse I). Das Dentalimplantat 10 weist im dargestellten Fall nicht nur einen in seiner Form im wesentlichen an den Zahn 1 bzw. an die Alveolenwand 7 angepassten Wurzelbereich 11 auf sondern auch einen an die Zahnkrone 5 angepassten Kronenbereich 12. Das Dentalimplantat 10 ist beispielsweise einstückig und besteht aus Titan, wobei der Kronenbereich mit einer nicht dargestellten Keramikschicht überzogen ist und die Oberfläche des Wurzelbereiches 11 mindestens bereichsweise für eine osseointegrative Wirkung ausgerüstet sein kann, aber mindestens biokompatibel oder knochenfreundlich ist. Anstelle des Kronenbereichs 11 kann das Dentalimplantat auch ein Abutment oder ein Mittel zur Befestigung eines Abutments, einer Krone, einer Brücke oder einer Zahnprothese aufweisen.

Der sich gegen das distale Ende verjüngende Wurzelbereich 11 des Dentalimplantates 10 weist stufenartige Querschnittsreduktionen 13 auf, deren Aussenkanten als gegen den distalen Endbereich gerichtete Schneidekanten 14 ausgebildet sind und die sich bei der Implantation in die Alveolenwand eingeschnitten haben. Zwischen den stufenartigen Querschnittsreduktionen 13 bleibt der Querschnitt des Implantates gegen das distale Implantatsende hin im wesentlichen konstant oder verkleinert sich kontinuierlich. Zwischen den stufenartigen Querschnittsreduktionen ist das Implantat in Bereichen 15 durch das thermoplastische Material mit dem Knochengewebe der Alveolenwand 7 verbunden. Wie bereits weiter oben angesprochen, werden diese Verbindungen während der Implantation erstellt. Dabei wird das thermoplastische Material mittels auf das Implantat wirkender mechanischer Vibrationen verflüssigt und in Unebenheiten und Poren der Alveolenwand gepresst, wo es nach der Wiederverfestigung formschlüssig und/oder stoffschlüssig verankert bleibt.

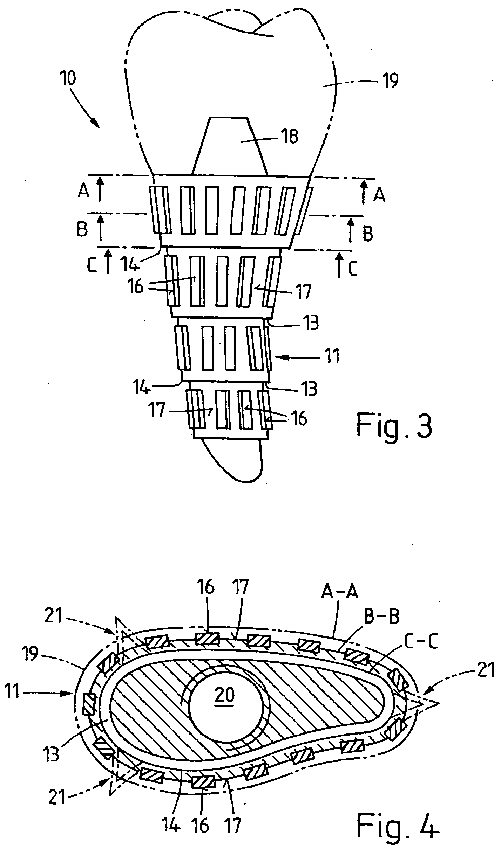

**Figur 3** zeigt ein ähnliches, individuelles Dentalimplantat 10 wie Figur 2, das aber noch nicht implantiert ist. Am Wurzelbereich 11 dieses Implantats sind die Schneidekanten 14 bzw. die stufenartigen Querschnittsreduktionen 13 gut sichtbar sowie zwischen diesen angeordnete Oberflächenbereiche 16 aus dem thermoplastischen Material, die über dazwischenliegende Oberflächenbereiche 17 vorstehen. Die Oberflächenbereiche 17 sind biokompatibel, vorteilhafterweise osseointegrativ ausgerüstet. Wenn das thermoplastische Material resorbierbar ist, ist vorteilhafterweise die ganze Oberfläche des Wurzelbereichs 11 osseointegrativ ausgerüstet.

Die Form des Wurzelbereiches 11 ist mindestens teilweise an die Form der zu ersetzenden, natürlichen Zahnwurzel oder an einen mechanisch relevanten Teil dieser Zahnwurzel bzw. an die Form der entsprechenden Alveolenwand angepasst, das heisst, sie weist generell dieselbe Konusform mit mindestes teilweise nicht runden Querschnitten und/oder ohne geradlinige Konusachse auf. Der Wurzelbereich 11 weist aber gegenüber der natürlichen Zahnwurzel bzw. Alveolenwand die stufenartigen Querschnittsreduktionen 13 auf, deren Kanten mindestens teilweise als Schneidekanten 14 ausgebildet sind, und die über die osseointegrativen Oberflächenbereiche 17 vorstehenden Oberflächenbereiche 16 aus dem thermoplastischen Material. Die Oberflächenbereiche 16 aus dem thermoplastischen Material sind dabei derart angeordnet und dimensioniert, dass das sich während der Implantation verflüssigende Material möglichst wenig auf die osseointegrativen Oberflächenbereiche 17 gedrückt wird, so dass diese unmittelbar nach der Implantation ihre osseointegrative Wirkung entfalten können.

**Figur 4** zeigt drei Querschnitte (etwa Schnittlinien A-A, B-B und C-C in Figur 3) durch ein erfindungsgemässes Implantat, das beispielsweise etwa dem Dentalimplantat der Figur 3 entspricht. Aus dem Querschnitt B-B ist klar ersichtlich, wie die Oberflächenbereiche 16 aus dem thermoplastischen Material über die osseointegrativen Oberflächenbereiche 17 vorstehen.

Wie bereits eingangs erwähnt und wie in der Figur 4 strichpunktiert dargestellt, kann ein erfindungsgemässes Implantat auch furchende oder schneidende, sich im wesentlichen axial erstreckende Strukturen 21 aufweisen, die derart dimensioniert sind, dass sie bei der Implantation in die Kavitätswand eindringen. Solche Strukturen geben dem Implantat eine zusätzliche Komponente primärerer Stabilität, insbesondere bezüglich Torsionskräften, und koppeln auch nach der Osseointegration auf das Implantat wirkende Torsionskräfte in das Knochengewebe ein.

Es zeigt sich, dass gute Resultate erzielt werden können, wenn der Wurzelbereich 11 eines Dentalimplantates gemäss Erfindung wie folgt dimensioniert wird:
- Die Querschnitte des Wurzelbereichs 11 sind in etwa so gross wie entsprechende Querschnitte durch die entsprechende Alveole (Zahnwurzel mit Zahnhaut). Die Schneidekanten 14 und gegebenenfalls die stufenartigen Querschnittsreduktionen 13 und axial verlaufende, furchende Strukturen 21 sowie die Oberflächenbereiche 16 mit dem thermoplastischen Material ragen über diese Querschnitte hinaus.
- Die axialen Abstände zwischen benachbarten, stufenartigen Querschnittsreduktionen 13 sind zum einen bedingt durch die Tiefe der Stufen sowie durch die lokale Steilheit des Wurzelbereiches, zum andern kann es aber vorteilhaft sein, vor allem nach proximal die Stufen zu verstärken, resp. die Abstände zu verkleinern und die Schneidekanten gegebenenfalls etwas ausladend zu gestalten, derart, dass sie tiefer in die Alveolenwand eingreifen und dadurch das Implantat optimal zu verankern.
- Die stufenartigen Querschnittsreduktionen 13 haben, vorgegeben durch den zur Verfügung stehenden Raum zwischen zwei Zähnen, eine Tiefe von maximal 1 mm, vorteilhafterweise von 0,1 bis 0,5 mm. Wenn sie weiter als ca. 0,3 mm über das Mass der Alveolenwand vorstehen, ist es empfehlenswert, in der Alveolenwand vor der Implantation entsprechende Schultern zu erstellen.
- Die Oberflächenbereiche 16 mit dem thermoplastischen Material ragen um 0,05 bis 2 mm (vorteilhafterweise 0,2 bis 1 mm) über die daneben liegenden Oberflächenbereiche 17.
- Die Oberflächenbereiche 16 aus dem thermoplastischen Material nehmen vorteilhafterweise 10 bis 50% der Gesamtoberfläche des Wurzelbereichs 11 ein und sind vorteilhafterweise sich axial erstreckend zwischen Oberflächenbereichen 17 angeordnet.

Entsprechend dem Belastungskollektiv lassen sich die oben gemachten Angaben auch auf andere Implantate übertragen. Die Tiefe der stufenartigen Querschnittsreduktionen kann bei verfügbarer Knochenmasse durchaus erhöht werden, um zum einen der Steilheit der Kavität zu entsprechen und zum anderen die Kräfte in dem Sinne optimal einleiten zu können, dass der Knochen ausreichend stimuliert wird, ohne lokal überbelastet zu werden. Dies bedeutet, dass die durch die Schneidekanten und die stufenartigen Querschnittsreduktionen übertragenen Beanspruchungen, im Mittel nach erreichter Osseointegration im Knochengewebe keine Dehnungen von mehr als 0.5% aber über 0.05% induzieren sollten.

Solche anderen Implantate sind beispielsweise an eine epi-, meta- und diaphysäre Geometrie bzw. an eine in dieser Geometrie erstellbare oder vorhandene Kavität angepasste Schäfte von Gelenkprothesen (Gelenkimplantat z.B. für Hüftgelenk-, Kniegelenk- oder Fingergelenkprothesen), die in entsprechend vorbereitete Röhrenknochen implantiert werden, oder es sind Implantate, die für die Reparatur von Defektstellen vorgesehen sind (beispielsweise Defektstellen im Schädel- oder Kieferbereich oder durch Tumorerkrankungen entstandene Defektstellen in irgend einem Knochenbereich). Es ist auch denkbar, die Erfindung auf Replika von bestehenden Implantaten anzuwenden, wobei in einer Revisionsoperation ein bestehendes Implantat mit nur minimalem Verlust an vitalem Knochengewebe durch ein an das bestehende Implantat bzw. an eine durch Entfernung des bestehenden Implantates entstehende Kavität angepasstes Individualimplantat ersetzt wird.

Die Oberflächenbereiche 16 aus dem thermoplastischen Material weisen vorteilhafterweise Energierichtungsgeber auf, das heisst, diese Bereiche laufen gegen aussen zu Kanten oder Spitzen zu oder sie weisen ein Muster von Höckern auf. Diese Energierichtungsgeber führen zu Spannungskonzentrationen, wenn das im Knochengewebe positionierte Implantat mit mechanischen Vibrationen angeregt wird, und stellen sicher, dass das thermoplastische Material sich an der Grenzfläche zum Knochenmaterial zu verflüssigen beginnt und dass ein thermoplastisches Material überhaupt verflüssigt werden kann.

Das thermoplastische Material ist vorteilhafterweise derart ausgewählt und am Implantat angeordnet, dass bei der Anwendung der mechanischen Vibration das Implantat als Ganzes akustisch anregbar ist, das heisst resonatorähnlich funktioniert, dass also die mechanischen Schwingungen im Innern des Implantates, also insbesondere an der Berührungsfläche zwischen nicht thermoplastischem Material und thermoplastischem Material oder im Innern des thermoplastischen Materials nicht relevant gedämpft werden. Dadurch wird erreicht, dass das thermoplastische Material an den Implantatoberflächen, insbesondere im Bereich der mit dem Knochengewebe in Berührung stehenden Energierichtungsgeber verflüssigt wird. Für eine geringe Dämpfung innerhalb des thermoplastischen Materials wird vorteilhafterweise ein Material mit einem Elastizitätsmodul von mindestens 0,5 GPa gewählt. Für die Vermeidung von Energieverlusten an den Grenzflächen zwischen den beiden Materialien wird das thermoplastische Material vorteilhafterweise starr und möglichst grossflächig mit dem nicht thermoplastischen Material verbunden.

Es zeigt sich, dass das thermoplastische Material bei der Implantation mittels Ultraschall etwa bis in eine Tiefe von zwei trabekulären Kammern in das Knochengewebe eingepresst werden kann, das heisst in eine Tiefe einer Grössenordnung von ca. 0,2 bis 1 mm. Um eine derartige Eindringtiefe zu erreichen, muss das thermoplastische Material in einer entsprechenden Menge vorliegen und muss zwischen den Oberflächenbereichen aus dem thermoplastischen Material und der Kavitätswand eine entsprechende, durch Übermass erwirkte, radiale Druckkraft herrschen.

Wie aus den Figuren 2, 3 und 4 zu ersehen ist, kann das Dentalimplantat zusätzlich zum Wurzelbereich 11 beispielsweise einen Kronenbereich 12 aufweisen (Figur 2) oder einen Konus 18 zum Montieren einer künstlichen Krone 19 (Figur 3) oder ein Mittel (beispielsweise Sackloch 20 mit Innengewinde, Figur 4) zur Befestigung eines Konus, eines Abutments oder einer Befestigungsvorrichtung für eine Brücke oder eine Zahnprothese. Solche Aufbauten sind aus dem Stande der Technik bekannt.

**Figuren 5A und 5B** zeigen in einem axialen Teilschnitt je eine Schneidekante 14 eines erfindungsgemässen Implantates in einem etwas grösseren Massstab, wobei das Implantat mit dem proximalen Endbereich gegen oben und dem distalen Endbereich gegen unten dargestellt ist. Die gegen den distalen Endbereich (in den Figuren gegen unten) gerichtete Schneidekante 14 weist einen Keilwinkel β auf, der kleiner ist als 90° (vorteilhafterweise 45° bis 80°) und ist relativ zur Implantatsachse etwas ausladend ausgestaltet, was ihr je nach Ausgestaltung der Ausladung gegenüber einer zur Implantatsachse I parallel verlaufenden Kavitätswand K (Bohrung) einen Freiwinkel α (Figur 5A) oder einen Freiraum a (Figur 5B) gibt, durch den Reibung an der Kavitätswand und dadurch erzeugte Wärme reduziert wird. Der Freiwinkel α ist dabei vorteilhafterweise klein (z.B. 1° bis 15°) und der Freiraum hat beispielsweise ein Tiefe von 0,1 bis 0,3 mm). Damit die Schneidekante 14 schneidend und spanbildend wirken kann, ist der Schnittwinkel, der α + β beträgt, kleiner als 90° oder der Winkel γ zwischen der Spanfläche 22 und der Implantatsachse 1 ist kleiner als 90°. Der durch die Schnittkante von der Kavitätswand abgehobener Span S wird in einen die Spanfläche 22 bildenden und als Spanraum 23 dienenden Hinterschnitt gestossen. Je nach Grösse des Spanraumes 23 wirkt die Schneidekante 14 nicht nur schneidend sondern auch verdichtend auf das Knochengewebe.

Wird ein Implantat mit einer Schneidekante 14 ähnlich wie die in den Figuren 5A und 5B dargestellten Schneidekanten 14 in einer leicht konischen Kavität (Bohrungswand K nicht parallel zur Implantatsachse) implantiert, erübrigt sich die Ausladung der Schneidekante 14; der Freiwinkel α ist dann z.B. gleich dem Winkel zwischen der Kavitätswand K und der Implantatsachse I.

**Figur 6** zeigt wiederum in einem axialen Teilschnitt eine Serie von in Implantationsrichtung (in der Figur von oben nach unten) hintereinander angeordneten Schneidekanten 14, 14' und 14", die ähnlich ausgestaltet sind wie die in Figur 5A dargestellte Schneidekante. Die Distanz zwischen den Schneidekanten und der Implantatsachse I nimmt in Implantatsrichtung ab, so dass die Schneidekanten nacheinander auf eine parallel zur Implantatsachse I verlaufende Kavitätswand K (K' vor der Einwirkung der Schneidekanten) spanbildend wirken können. Offensichtlich sind also auch in einem derartigen Falle die Schneidekanten 14, 14' und 14" mit minimalen Querschnittsreduktionen verbunden. Die Tiefen (d) dieser Querschnittsreduktionen sind aber im Unterschied zu einem konischen Implantat (z.B. gemäss Figuren 2 und 3) nicht von der allgemeinen Form von Kavität bzw. Implantat abhängig sondern können optimal für eine gute Spanbildung bzw. gute Verankerung des Implantats ausgelegt werden. Für ein in einer entsprechenden Bohrung zu implantierendes Dentalimplantat sind die genannten Tiefen d vorteilhafterweise nicht grösser als 0,3 mm.

Wenn die Spanräume 23 für das gesamte Spanmaterial nicht genügend gross sind, wird das Spanmaterial darin verdichtet. Um eine zu grosse Verdichtung zu vermeiden kann auch beispielsweise mittels Kanälen 25 mindestens ein Teil des Spanmaterials aus den Spanräumen entfernt werden beispielsweise durch Absaugen oder durch Spülen, wobei für eine Spülung durch eine entsprechende Formgebung des Implantats dafür gesorgt wird, dass das ausgespülte Material (Spanmaterial und Spülmittel) zwischen Kavitätswand und Implantat aus der Kavität abfliessen kann.

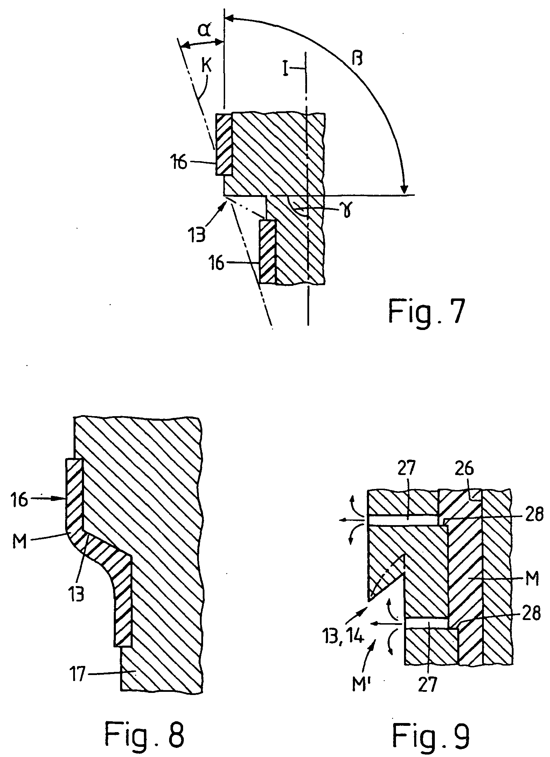

**Figur 7** zeigt wiederum als axialen Teilschritt eine stufenartige Querschnittsreduktion 13, die in diesem Falle keine Schneidekante aufweist (Schnittwinkel α + β in einer Bohrung mit einer zur Implantatsachse I parallelen Kavitätswand gleich 90°, in einer konischen Kavität mit Kavitätswand K grösser als 90°; Winkel γ gleich oder grösser als 90°) und deren Kante deshalb höchstens schabend auf die Kavitätswand wirken kann. Derartige Querschnittsreduktionen 13 können an konischen Implantaten zusätzlich zu Querschnittsreduktionen mit Schneidekanten vorgesehen werden. In Figur 7 sind auch die Oberflächenbereiche 16 mit dem verflüssigbaren Material M sichtbar, wobei das verflüssigbare Material in Vertiefungen angeordnet ist und über die umgebenden Oberflächenbereiche vorsteht. Wenn eine Querschnittsreduktion 13 keine Schneidekante aufweist, kann das verflüssigbare Material bzw. eine dafür vorgesehene Vertiefung auch darüber hinweg verlaufen, wie das in der Figur 8 in einem weiteren axialen Schnitt durch eine solche Querschnittsreduktion 13 dargestellt ist.

**Figur 9** zeigt wiederum in einem axialen Teilschnitt ein erfindungsgemässes Implantat, das einen Innenhohlraum 26 aufweist, in dem das verflüssigbare Material M vor der Implantation positioniert ist oder positioniert wird, und Öffnungen 27 durch die das verflüssigbare Material während der Implantation in verflüssigtem Zustand (M') an die Implantatsoberfläche gepresst wird, wo es erst dann Oberflächenbereiche des Implantates und sofort oder nach der Wiederverfestigung auch eine Verankerung zwischen Knochengewebe und Implantat bildet. Der Innenhohlraum 26 ist vorteilhafterweise mit Energierichtungsgebern 28, z.B. in Form von kantigen Stufen versehen, um den Energiebedarf für eine optimale Verflüssigung des vorgelegten verflüssigbaren Materials zu minimieren und eine möglichst tiefe Viskosität zu erreichen.

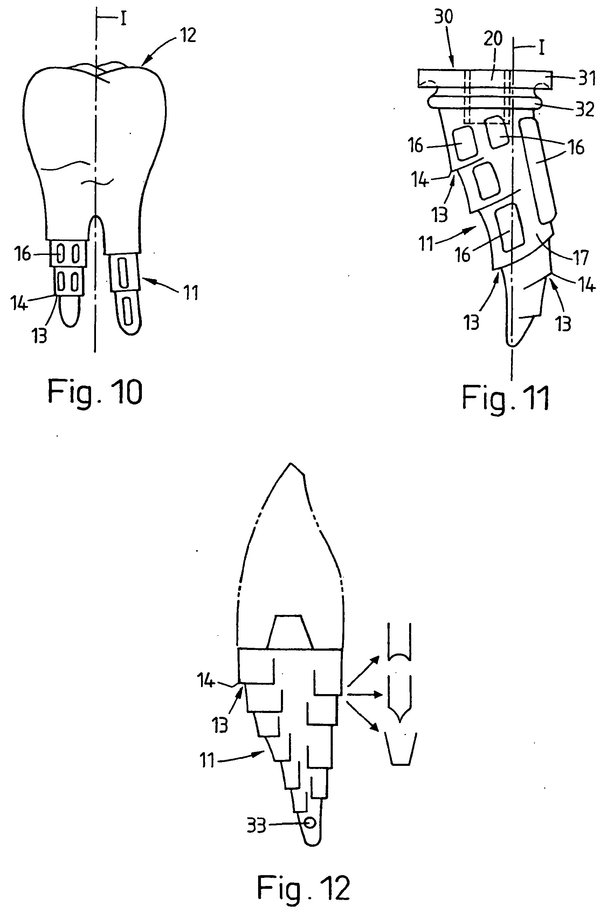

**Figur 10** zeigt als weiteres Beispiel eines erfindungsgemässen Implantates ein individuelles Dentalimplantat, das wie ein Backenzahn einen mehrteiligen Wurzelbereich 11 aufweist. Dieser Wurzelbereich 11 muss nicht unbedingt den gesamten, natürlichen Wurzelbereich ersetzen, sondern kann auf dessen mechanisch relevante und/oder extrahierbare Teile beschränkt sein. Auch in diesem Falle ist es möglich, das Implantat unmittelbar nach der Extraktion des zu ersetzenden Backenzahnes zu implantieren und unmittelbar nach der Implantation zu belasten. Das Implantat kann also einen Kronenbereich 12 aufweisen, der beispielsweise der Krone des extrahierten Zahnes nachgebildet ist.

**Figur 11** zeigt ein weiteres, individuelles Dentalimplantat gemäss Erfindung, das wiederum einen Wurzelbereich 11 mit stufenartigen Querschnittsreduktionen 13, die mindestens teilweise mit Schneidekanten 14 ausgerüstet sind, aufweist. Diese Querschnittsreduktionen 13 sind axial unregelmässig über den Wurzelbereich 11 verteilt und sie verlaufen nicht vollständig um diesen herum und eher schief zur Implantatsachse I. Entsprechend unregelmässig sind auch die Oberflächenbereiche 16 aus thermoplastischem Material und die osseointegrativen Oberflächenbereiche 17 angeordnet. Das Implantat weist ferner einen Abutment-artigen, proximalen Teil 30 auf, der nach der Implantation aus dem Kieferknochen in den Bereich des Zahnfleisches ragt und beispielsweise zur Befestigung weiterer Aufbauten mit einem Sackloch 20 mit Innengewinde ausgerüstet ist. Der Abutmentteil 30 weist einen Kragen 31 mit einer schneidenförmig hinterschnittenen Unterkante auf. Mit dieser Unterkante stützt sich der Abutmentteil 30 nach der Implantation leicht einschneidend auf der Oberfläche des Kieferknochens ab. Unmittelbar unter dem Kragen 31 ist ein Ring 32 aus thermoplastischem Material angeordnet, der durch Verflüssigung während der Implantation das Implantat in der äussersten Schi cht des Kieferknochens verankern soll. Dieser Ring 32 besteht vor allem für einen älteren Patienten vorteilhafterweise aus einem nicht resorbierbaren Thermoplasten, derart, dass er zusätzlich zu seiner Verankerungsfunktion eine Dichtungsfunktion zwischen dem Knochengewebe und dem darüber angeordneten, gegebenenfalls nicht sehr dicht um das Implantat anschliessenden Zahnfleisch übernehmen kann.

Kragen 31 und Ring 32 können funktionsgemäss auch unabhängig voneinander angewendet werden. Ferner können sie einzeln oder in Kombination auch für standardisierte Dentalimplantate und auch für andere als Dentalimplantate allgemein für eine Verankerung in einer Knochenoberfläche und für eine Abdichtung der Kavität gegenüber dem Knocheninneren angewendet werden.

Dadurch, dass durch die nicht kreiszylindrische und nicht kreiskegelförmige und im Falle eines Individualimplantates überhaupt nicht rotationssymmetrische Form des erfindungsgemässen Implantates ist die Orientierung des Implantates in der Kavität genau definiert. Aus diesem Grunde ist es auch möglich, den Kragen 31 nicht, wie in der Figur 11 dargestellt, quer zur Implantatsachse, eben und rund (rotationsinvariant) auszugestalten sondern an den natürlichen Zahn angepasst beispielsweise etwa oval und an den natürlichen gebogenen Verlauf eines Kieferkammes angepasst, sogenannt skallopiert (muschelförmig).

**Figur 12** zeigt ein weiteres Implantat gemäss Erfindung, das wiederum beispielhaft als Dentalimplantat ausgestaltet ist und einen Wurzelbereich 11 aufweist mit daran angeordneten, stufenartigen Querschnittsreduktionen 13, die mindestens teilweise als Schneidekanten 14 ausgebildet sind und die in diesem Falle auf Teile des Implantatumfangs beschränkt sind, so dass sie sich schuppenförmig von der übrigen Implantatsoberfläche abheben. Diese schuppenartigen Strukturen können wie am Wurzelbereich 11 dargestellt eine im wesentlichen rechteckige oder quadratische Form haben und ihre um den Umfang verlaufenden Kanten (Unterkanten) können wie weiter oben beschrieben (Figuren 5 bis 7) stumpf oder schneidend ausgestaltet sein. Dasselbe gilt für die im wesentlichen axial verlaufenden Kanten der schuppenförmigen Strukturen, die, wenn schneidend ausgerüstet, als axial verlaufende furchende oder schneidende Strukturen funktionieren.

Rechts vom Wurzelbereich 11 sind in der Figur 12 weitere beispielhafte Formen von stufenartigen Querschnittsreduktionen 13 mit schuppenartigen Formen dargestellt. Diese können beispielsweise in axialer Richtung konkave (seitlich oder mittig spitz zulaufende) oder konvexe (nicht dargestellt) Unterkanten oder schief gegen die Unterkante zulaufende axiale Kanten aufweisen. Auch in radialer Richtung können diese Unterkanten und Bereiche über den Unterkanten konvex oder konkav gebogen oder eben sein, die "Schuppe" also die Form eines Konus oder Hohlkonus aufweisen oder im wesentlichen eben sein.

Es ist auch möglich, den Wurzelbereich 11 des individuellen Dentalimplantats in an sich bekannter Weise mit einer oder mehreren durchgehenden Öffnungen 33 zu versehen. Solche Öffnungen werden während der Osseointegrations-Phase von Knochengewebe durchwachsen.

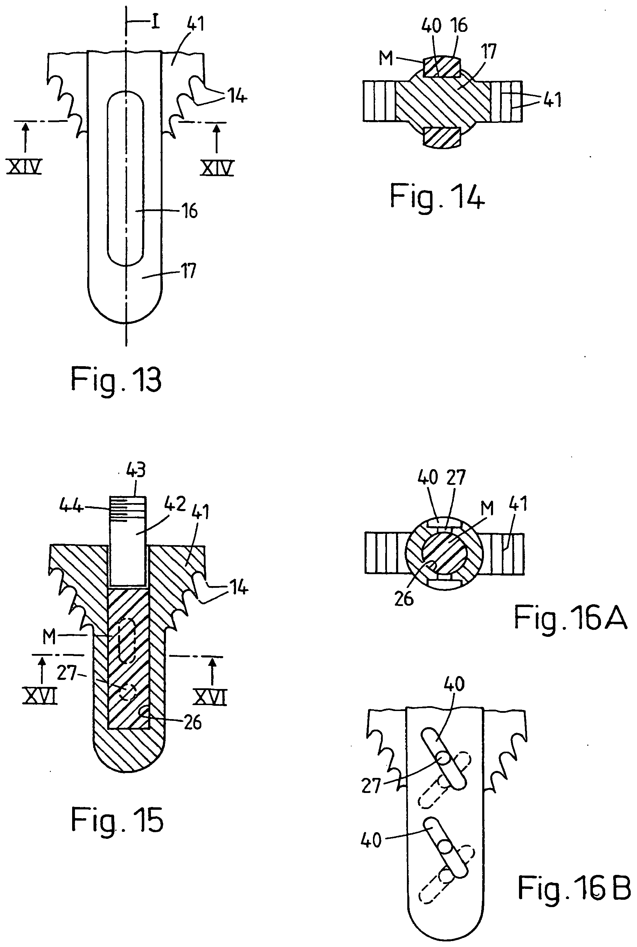

**Figuren 13 und 14** zeigen eine weitere Ausführungsform eines erfindungsgemässen Implantates, das beispielsweise als Dentalimplantat anwendbar ist. Figur 13 zeigt das Implantat in Seitenansicht, Figur 14 quer zur Implantatsachse I geschnitten (Schnittlinie XIV-XIV in Fig. 13). Das Implantat ist im wesentlichen zylindrisch und ist ausgelegt für die Implantierung in einer zylindrischen Bohrung. Das Implantat weist auf einander relativ zur Implantatsachse gegenüberliegenden Seiten Oberflächenbereiche 16 aus einem thermoplastischen Material M auf, wobei das thermoplastische Material in Vertiefungen 40 (hier als in axialer Richtung geschlossene Nuten) angeordnet ist und gegenüber den benachbarten Oberflächenbereichen 17 vorsteht. Auf dem Implantatsumfang zwischen den Oberflächenbereichen 16 mit dem thermoplastischen Material weist das Implantat gegen sein proximales Ende Anformungen 41 auf, die die Schneidekanten 14 tragen. Diese verlaufen im wesentlichen quer zur Implantatsachse und weisen wie in der Figur 6 dargestellt in Implantationsrichtung kleiner werdende Abstände zur Implantatsachse I auf. Diese Abstände variieren von Schneidekante zu Schneidekante um etwa 0,3 mm (für Dentalimplantat), derart, dass das Implantat ohne entsprechende Vorformung der Kavität implantierbar ist. Das heisst mit anderen Worten, die Schneidekante 14 sind derart ausgelegt, dass die je vorlaufende Schneidekante die Kavitätswand durch Abheben eines Spans derart verändert, dass die folgende Schneidkante optimal und wieder spanbildend auf diese wirken kann. Die quer zur Implantatsachse gemessenen Abstände zwischen in axialer Richtung aufeinander ausgerichteten Schneidekanten können für grössere Implantate als Dentalimplantate auch grösser sein als 0,3 mm.

Das Implantat gemäss Figuren 13 und 14 wird also in eine rotations-invariante Kavität (kreiszylindrische Bohrung) implantiert und ist nach der Implantation trotzdem durch seine nicht rotierbare Form gegen rotative Belastungen stabilisiert. Gegenüber einem schraubenförmigen Implantat hat das vorliegende Implantat den Vorteil, dass es in einer genau vorbestimmten Rotationsposition implantierbar ist und aus diesem Grunde auch andere als rotations-invariante Aufbauten tragen kann, z.B. einen skallopierten Kragen, eine Krone etc.

Es ist für das Implantat gemäss Figuren 13 und 14 keine Bedingung, dass die für das thermoplastische Material vorgesehenen Vertiefungen 40 axial verlaufende Nuten sind. Sie können insbesondere auch leicht spiralig verlaufende Nuten sein, die besser fähig sind, Torsionskräfte aufzunehmen. Ferner kann das Implantat gemäss Figuren 13 und 14 auch für die Implantation in einer Stufenbohrung oder einer Kavität mit einem konisch zulaufenden inneren Ende zusätzlich zu den Schneidekanten auch stufenförmige Querschnittsreduktionen (nicht dargestellt) aufweisen, über die Vertiefungen 40 und das darin angeordnete, verflüssigbare Material M sich fortsetzen kann, wie dies in Figur 8 dargestellt ist.

**Figuren 15, 16A und 16B** zeigen ein weiteres erfindungsgemäss es Implantat (Fig. 15: axialer Schnitt; Fig. 16A: Schnitt quer zur Implantatsachse mit Schnittlinie XVI-XVI; Fig. 16B: Seitenansicht), beispielsweise ein Dentalimplantat, das in seiner Form dem Implantat gemäss Figuren 13 und 14 im wesentlichen entspricht, das aber einen inneren Hohlraum 26 aufweist und Öffnungen 27, die den inneren Hohlraum 26 mit der Aussenoberfläche des Implantats verbinden und beispielsweise im wesentlichen rund oder schlitzförmig sind. Die Öffnungen 27 münden in die Vertiefungen 40, deren Grund zur besseren Haftung des verflüssigbaren Materials beispielsweise aufgeraut ist. Das verflüssigbare Material M, das in diesem Falle ein thermoplastisches oder thixotropes Material sein kann, wird vor oder während der Implantation im inneren Hohlraum 26 positioniert und mit Hilfe von mechanischer Vibration mindestens teilweise verflüssigt und durch die Öffnungen 27 in die Vertiefungen 40 gepresst, die zwischen Implantat und Kavitätswand Taschen bilden, in die das verflüssigte Material durch die Vertiefungsform geführt eingepresst wird und dadurch mit der Kavitätswand in intensiven Kontakt kommt. Wie aus der Figur 16A ersichtlich ist, können, insbesondere für den Fall des Implantates mit Hohlraum 26 die Vertiefungen 40 auch als spiralig um das Implantat verlaufende Nuten ausgebildet sein, wodurch das Implantat durch das verflüssigbare Material noch besser gegen Drehung in der Kavität stabilisiert wird.

Vorteilhafterweise wird das Implantat gemäss Figuren 15, 16A und 16B ohne Verflüssigung des verflüssigbaren Materials M implantiert, das heisst in seine endgültige Position in der Kavität gebracht, wozu es mit einem üblichen Werkzeug eingeschlagen oder mit einem mechanisch oszilierenden Element (z.B. Sonotrode eines Ultraschallgerätes) eingepresst wird. Die Implantatsposition wird dann überprüft und gegebenenfalls noch bezüglich Tiefe und Rotationsposition geringfügig ajustiert und erst dann wird das verflüssigbare Material mit mechanischen Schwingungen beaufschlagt und gegen das distale Implantatsende gepresst, wodurch es verflüssigt wird und durch die Öffnungen 27 austritt, die Vertiefungen 40 füllt und in das umliegende Knochengewebe eindringt. Damit das Implantat für die genannte Überprüfung und gegebenenfalls Ajustierung seiner Position genügend stabil in der Kavität sitzt, ist es für diesen Fall gegebenenfalls vorteilhaft, das Implantat gegenüber der Kavität leicht überdimensioniert auszulegen, derart, dass nicht nur die Schneidekanten im Knochengewebe eingeschnitten werden sondern dass das Implantat durch Pressfit in der Kavität gehalten wird.

Für die Verflüssigung des verflüssigbaren Materials kann eine auf den Querschnitt des Hohlraumes 26 abgestimmte Sonotrode verwendet werden oder ein Kolben 42, der ein Bestandteil des Implantates ist. Auf dem proximalen Ende 43 des Kolbens 42 wird eine Sonotrode zur Einkoppelung von mechanischen Schwingungen aufgesetzt. Der Kolben 42 ist derart ausgelegt, dass er mit fortschreitender Verflüssigung und Verdrängung des verflüssigbaren Materials aus dem Hohlraum 26 in diesen eindringt, bis sein proximales Ende 43 in den Bereich der Öffnung des Hohlraumes 26 kommt. Dabei besteht der Kolben 43 beispielsweise aus Titan und ist im Bereich des proximalen Endes 43 mit einem Feingewinde 44 ausgerüstet, das beim Einschub in den Hohlraum 26 mit der ebenfalls aus Titan bestehenden Hohlraumwand kaltverschweisst wird. Dadurch wird die proximale Öffnung des Hohlraumes 26 dicht verschlossen, wodurch für ein Dentalimplantat die notwendige Abdichtung zwischen Mundhöhle und Knochengewebe garantiert ist. Wenn das verflüssigbare Material resorbierbar ist, wird das Knochengewebe nach der Implantation dieses Material sukzessive ersetzen, das heisst in die Öffnungen 27 und den Hohlraum 26 einwachsen, wobei es in diesem Falle um so wichtiger ist, dass der Hohlraum 26 gegen die Mundhöhle dicht verschlossen ist.

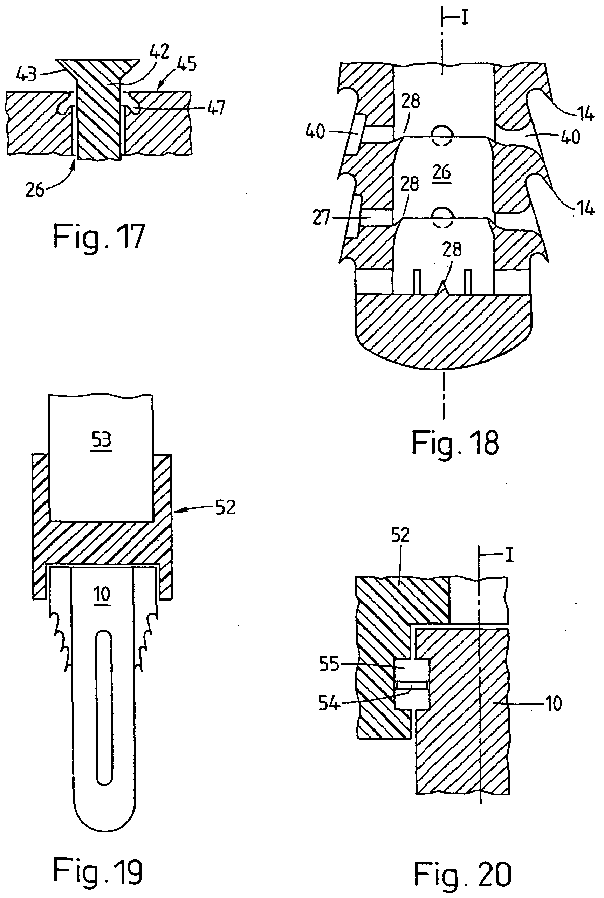

**Figur 17** zeigt wie Figur 15 in einem axialen Teilschnitt einen Kolben 42, der zur Verdrängung des verlüssigbaren Materials aus dem Hohlraum 26 in dessen proximaler Öffnung positioniert ist und derart ausgelegt ist, dass sein proximales Ende 43 die proximale Fläche 45 des Implantates erreicht, wenn genügend verflüssigbares Material aus dem Hohlraum 26 durch die dafür vorgesehenen Öffnungen 27 auf die Implantatsaussenfläche gepresst worden ist. Das proximale Kolbenende 43 ist konisch verbreitert und der Kolben 42 besteht im vorliegenden Fall aus einem thermoplastischen Material, z.B. aus PEEK. Wenn die Kante um die proximale Öffnung des Hohlraumes 26 mit dem oszillierenden, verbreiterten Kolbenende 43 in Berührung kommt, wirkt sie als Enegierichtungsgeber und bewirkt das thermoplastische Material verflüssigende Spannungskonzentrationen. Das verflüssigte Material dringt zwischen die Wand des Hohlraumes 26 und den Kolben 42 ein, wo vorteilhafterweise noch eine Dichtungsnut 47 vorgesehen ist, und schliesst damit zusammen mit dem Kolben 42 die proximale Öffnung des Hohlraumes 26 dicht ab.

**Figur 18** zeigt wiederum einen axialen Teilschnitt durch ein erfindungsgemässes Implantat mit innerem Hohlraum 26, der durch Öffnungen 27 mit der Aussenfläche des Implantates verbunden ist. Damit das verflüssigbare Material, das in diesem Falle ein Thermoplast ist, durch die Wirkung der mechanischen Schwingungen gezielt im Bereich der Öffnungen 27 verflüssigt wird, sind an den inneren Mündungen der Öffnungen 27 Energierichtungsgeber 28, beispielsweise in Form von scharfen um den Umfang des Hohlraumes 26 verlaufende Kanten, vorgesehen. Am distalen Ende des Hohlraumes 26 kann beispielsweise ein dornförmiger Energierichtungsgeber 28 vorgesehen sein. Ein im Hohlraum 26 vorgeschobenes und mit mechanischen Schwingungen beaufschlagtes Stück aus dem verflüssigbaren Material trifft auf die Energierichtungsgeber 28, die beim Kontakt mit dem oszillierenden Material Spannungskonzentrationen in diesem Material und eine örtliche Verflüssigung des Materials hervorrufen.

Ebenfalls aus der Figur 18 ersichtlich sind verschiedene Ausführungsformen von Öffnungen 27 und deren Mündungen in Vertiefungen 40. Die Öffnungen 27 haben beispielsweise einen runden (in der Figur oben) oder einen schlitzförmigen (in der Figur unten) Querschnitt und die Vertiefungen können durch eine Kante von den Öffnungen getrennt sein (in der Figur links) oder als Erweiterungen der Mündungen der Öffnungen ausgestaltet sein (in der Figur rechts). Auch Kombinationen der genannten Merkmale sind denkbar.

**Figur 19** zeigt am Beispiel eines Implantates gemäss Figuren 13 und 14 ein Übergangselement 52, das für die Implantation mittel mechanischer Schwingungen, insbesondere mittels Ultraschallschwingungen, geeignet ist. Das Übergangselement 52 ist implantatseitig an den proximalen Endbereich eines spezifischen, gegebenenfalls individuellen Implantats 10 angepasst und anregerseitig an eine vorteilhafterweise standardisierte Sonotrode 53, die Teil eines Ultraschallgerätes ist. Dabei ist vorteilhafterweise die implantatseitige und/oder die anregerseitige Verbindung des Übergangselements 52 als Spielpassung ausgelegt, das heisst als Verbindung mit Spiel in axialer Richtung und Führungsfunktion in radialer Richtung. Die je andere Verbindung kann eine starre Verbindung, z.B. Klemmpassung mit Reibschluss oder geschraubte Verbindung sein.

Das Übergangselement 52 besteht vorteilhafterweise aus einem Material (z.B. PEEK) mit geringer akustischer Dämpfung (hohes Elastizitätsmodul) und kann durch entsprechende Formgebung oder Materialwahl eine akustische Anpassung zwischen Sonotrode 53 und Implantat 10 bewirken. Das Übergangselement 52 kann zusätzlich zu seiner Interface-Funktion zwischen der standardisierten Sonotrodengeometrie und einer spezifischen Implantatsgeometrie auch eine akustische Anpassungsfunktion haben, es kann für Orientierungs- und Messzwecke entsprechende Markierungen tragen, es kann als vom Chirurgen leicht ergreifbarer, nicht direkt zum Implantat gehörender Teil dienen, durch den das Implantat, insbesondere, wenn es ein relativ kleines Dentalimplantat ist, besser handhabbar wird. Vorteilhafterweise wird das Übergangselement 52 herstellerseitig auf dem Implantat 10 positioniert und wird nach der Implantation entsorgt. In dieser Funktion kann es auch Teil der Implantatsverpakkung darstellen. Wenn das Übergangselement 52 aus einem transparenten Material besteht, kann es auch eine lichtleitende Funktion übernehmen, indem Licht von der Sonotrodenseite her in das Element eingekoppelt wird, um auf der Implantatseite gegebenenfalls in das Implantat eingekoppelt zur Beleuchtung der Kavität und der Implantation zu dienen.

Eine Verbindung mit Spielpassung zwischen Implantat 10 und Übergangselement 52 und/oder zwischen Übergangselement 52 und Sonotrode 53 (oder zwischen Sonotrode und Implantat, wenn kein Übergangselement verwendet wird) kann nur gegen das Implantat gerichtete, also das Implantat in die Kavität treibende, axiale Schwingungskomponenten auf das Implantat übertragen. Das Implantat aus der Kavität ziehende Schwingungskomponenten werden nicht übertragen. Es hat sich gezeigt, dass die Implantation mittels derart erzeugter Halbwellen vorteilhaft ist. Ein Grund dafür ist wahrscheinlich die Tatsache, dass Rückwärtsbewegungen des Implantates in der Kavität ausbleiben und damit die zwischen Kavitätswand und Implantat erzeugte Reibungswärme geringer ausfällt. Ein weiterer Vorteil der Spielpassung besteht darin, dass sie das Implantat akustisch von der Sonotrode und gegebenenfalls vom Übergangselement trennt und dass dadurch eine genaue akustische Abstimmung zwischen Anregerteilen und Implantat weniger wichtig wird.

Die Spielpassung wird beispielsweise realisiert durch eine Passung zwischen Implantat und Übergangselement, die wie eine Kapillare wirkt und in die unmittelbar vor der Implantation Flüssigkeit gegeben wird. Das in das Übergangselement gesteckte Implantat wird also, während es gegen oben gerichtet ist, auf der Sonotrode montiert und entpackt und dann wird zwischen dem proximalen Endbereich des Implantats und dem Übergangselement Flüssigkeit, beispielsweise Wasser, aufgebracht, das sich durch die Kapillarwirkung zwischen den beiden Teilen verteilt und diese genügend stark zusammenhält, dass das Implantat gegen unten gerichtet werden kann, ohne dass es aus der Passung fällt.

**Figur 20** zeigt in einem axialen Teilschnitt eine weitere Ausführungsform einer Spielpassung zwischen einem erfindungsgemässen Implantat 10 und einem Übergangselement 52 (oder zwischen dem Übergangselement und der Sonotrode oder zwischen dem Implantat und der Sonotrode); Diese besteht im wesentlichen aus einem Spannring 54, der in axial überdimensionierten, aufeinander ausgerichteten Ringnuten 55 von Implantat 10 und Übergangselement 52 positioniert ist und der aus einem Material besteht, das das Gewicht des Implantats halten kann, aber eine Zerstörung des Rings bzw. ein Ausfahren des Implantates aus der Passung mit wenig Kraftaufwand erlaubt. Weitere Varianten von Spielpassungen gehören zum Stande der Technik und sind im vorliegenden Fall in analoger Weise anwendbar.

Wie Figur 20 zeigt, braucht das Übergangselement 52 zwischen Sonotrode 53 und Implantat 10 nicht durchgehend zu sein. Sie kann ohne weiteres in diesem Zwischenbereich Öffnungen aufweisen oder andere geeignete Strukturen.

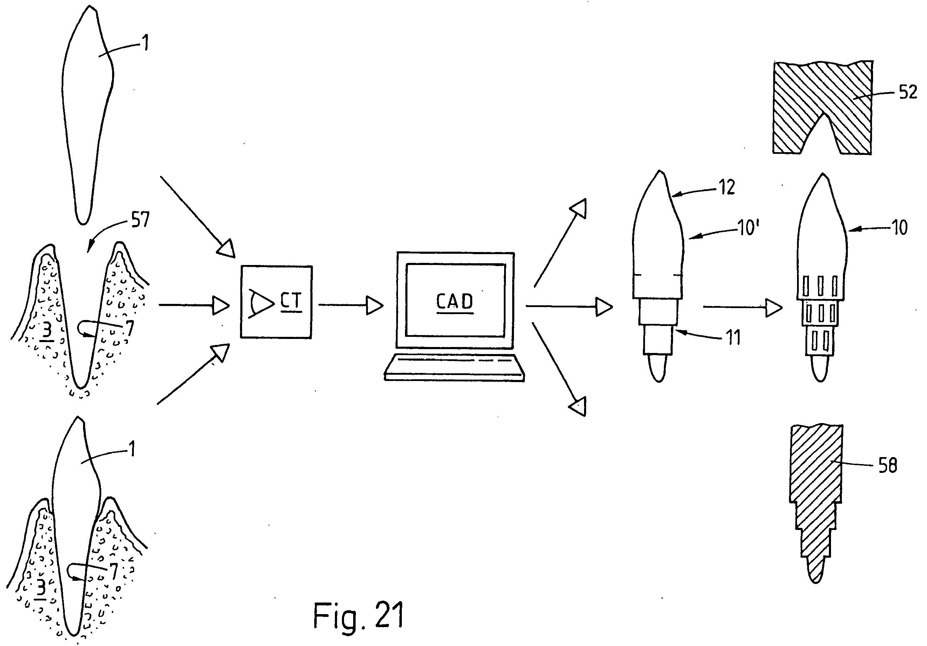

**Figur 21** illustriert das Verfahrens zur Herstellung eines Dentalimplantates 10 gemäss Erfindung. Dieses Verfahren weist im wesentlichen drei Schritte auf, die alle auf an sich bekannten Methoden beruhen. Diese Schritte sind:
- Vermessung: Ein zu ersetzender Zahn 1 und/oder die entsprechende Alveole 57, bzw. Alveolenwand 7 wird zur Erstellung eines beispielsweise dreidimensionalen Bildes vermessen. Die das Bild darstellenden Messdaten werden für eine Weiterverarbeitung bereitgestellt.
- Messdaten-Verarbeitung: Die das Bild darstellenden Messdaten werden insbesondere durch Addition von Schneidkanten und Strukturen aus verflüssigbarem Material, gegebenenfalls durch Addition eines Übermasses oder von furchenden oder schneidenden, axial verlaufenden Strukturen verändert. Wenn das Bild nicht ein dreidimensionales, vollständiges Bild ist, wird es vorgängig durch Erfahrungswerte bezüglich Implantatsform ergänzt. Die verarbeiteten Messdaten werden für eine Implantats-Herstellung bereit gestellt.
- Implantat-Herstellung: das Implantat wird anhand der verarbeiteten Messdaten gegebenenfalls in einer Mehrzahl von Herstellungsschritten hergestellt.

Für den Vermessungsschritt eigenen sich verschiedene Methoden, insbesondere die Methode der Computertomographie (CT) oder eine MRI-Methode (Magnetic Resonance Imaging), mit welchen Methoden beispielsweise für einen noch nicht extrahierten Zahn gleichzeitig ein Bild des Zahnes 1 und der Alveole 57 erstellt werden kann. Ein solches Verfahren erlaubt es, das Implantat vor der Extraktion des zu ersetzenden, natürlichen Zahnes herzustellen und in nur einer Session den zu ersetzenden Zahn zu extrahieren und an seiner Stelle das Implantat zu implantieren.

Es ist aber durchaus möglich, den extrahierten Zahn und/oder die Alveole 57 nach der Extraktion zu vermessen, wobei damit insbesondere Alveolenverformungen durch die Extraktion mitvermessen werden können.

Anstelle eines dreidimensionalen Bildes, für dessen Aufnahme komplexe Apparaturen zur Verfügung stehen müssen, ist es auch möglich, ein zweidimensionales Röntgenbild oder eine Mehrzahl solcher Bilder entsprechend auszumessen, wobei zur Erstellung eines dreidimensionalen Modells für das Implantat die Bilder anhand von entsprechenden Erfahrungswerten ergänzt werden.

Für den Schritt der Messdaten-Verarbeitung wird vorteilhafterweise ein CAD-System (computer aided design) verwendet, dem die Messdaten aus dem Vermessungsschritt zugeführt werden. Wenn Messdaten der Alveole 57 verfügbar sind, wird der Wurzelbereich des Implantates vorteilhafterweise anhand dieser Daten modelliert. Wenn nur Messdaten des zu ersetzenden Zahnes verfügbar sind, wird gegebenenfalls eine Erfahrungsdicke der Zahnhaut zugefügt. Für ein Implantat mit Hohlraum kann gegebenenfalls für einen Pressfit ein Übermass zugefügt werden. Ferner werden die seitlichen Oberflächen des Wurzelbereiches modifiziert durch Anfügen der Schneidekanten und der Oberflächenbereiche aus dem thermoplastischen Material und gegebenenfalls durch Osseointegrations-fördernde Strukturen. Für die Oberflächenbereiche aus dem thermoplastischen Material werden an einem Vorimplantat 10' gegebenenfalls Vertiefungen vorgesehen, in die Teile aus dem thermoplastischen Material vorteilhafterweise formschlüssig eingebracht werden können. Für die osseointegrativen Oberflächenbereiche werden beispielsweise entsprechende Oberflächenstrukturen vorgesehen.

Im Schritt der Messdaten-Verarbeitung können auch Daten erzeugt werden, aufgrund deren ein Übergangselement 52 herstellbar ist, wobei dieses an einen proximalen Endbereich des Implantates, beispielsweise an seinen Kronenbereich 12 möglichst genau angepasst ist. Ebensolche Daten können erzeugt werden für die Herstellung eines Bearbeitungswerkzeugs 58 oder eines Sets von Bearbeitungswerkzeugen, wobei diese Werkzeuge an den Wurzelbereich des Implantates angepasst sind (mit leichtem Untermass für ein Bearbeitungswerkzeug, bzw. stufenweise steigendem Untermass für eine Mehrzahl von Bearbeitungswerkzeugen). Das Bearbeitungswerkzeug 58 dient für die Vorbereitung der Alveolenwand vor der Implantation des Implantates.

Für den Schritt der Implantat-Herstellung wird vorteilhafterweise ein CAM-System (computer aided machining) verwendet, dem die Daten aus dem Schritt der Messdatenverarbeitung zugeführt werden. In diesem Schritt wird beispielsweise aus einem entsprechenden Titan-Rohling beispielsweise durch Fräsen, Schleifen oder Elektroerosion ein Vorimplantat 10' gefertigt. Aus diesem werden durch entsprechende Oberflächenbehandlung anschliessend die osseointegrativen Oberflächenbereiche erstellt und Teile aus dem thermoplastischen Material angebracht (durch Einschnappen, Aufkleben, Angiessen, durch Ultraschall etc.), wodurch das fertige Implantat 10 entsteht.

In im wesentlichen derselben Weise wie das Vorimplantat 10' werden auch das Übergangselement 52 und das oder die Bearbeitungswerkzeuge 57 für die Vorbereitung der Alveolenwand hergestellt.

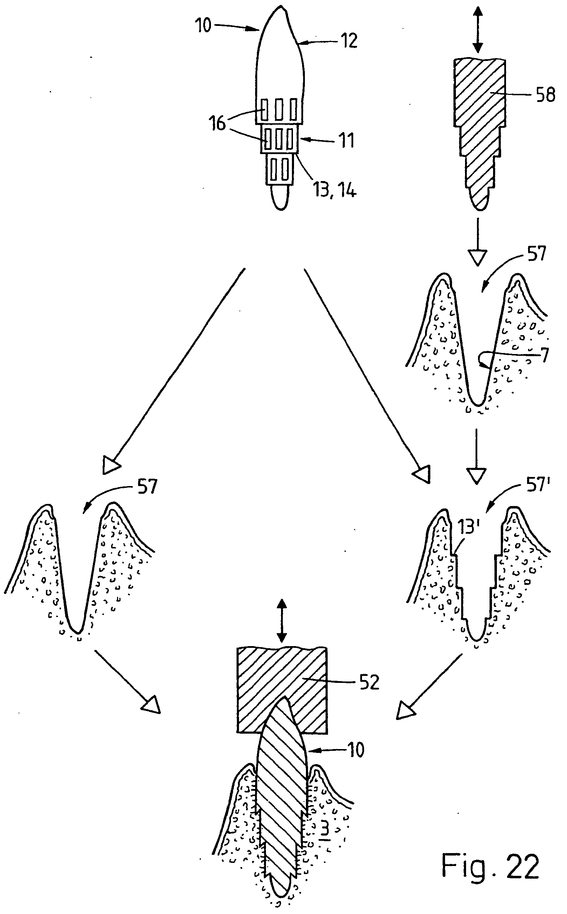

**Figur 22** illustriert das Verfahren zur Implantation eines Dentalimplantates gemäss Erfindung, wobei das gezeigte Implantat 10 zusätzlich zu einem erfindungsgemäss ausgerüsteten Wurzelbereich 11 auch einen Kronenbereich 12 aufweist, wobei beide diese Bereiche an die Form eines zu ersetzenden, natürlichen Zahnes angepasst sind. Der Wurzelbereich 11 des gezeigten Implantates 10 weist stufenartige Querschnittsreduktionen 13 mit Schneidekanten 14 und Oberflächenbereiche 16 aus einem thermoplastischen Material und gegebenenfalls axial verlaufende, furchende oder schneidende Geometrien (nicht dargestellt) auf.

Die Alveole 57 wird vor der Implantation ausgeräumt und kürettiert, wozu beispielsweise ein mit Ultraschall angetriebenes Werkzeug (nicht dargestellt) verwendet wird. Wenn die durch eine direkte Implantation entstehende Belastung des Knochengewebes tolerierbar ist, wird das Implantat in die derart vorbereitete Alveole 57 direkt implantiert (in Figur 22 links dargestellte Variante). Wenn die Belastung des Knochengewebes eher gering gehalten werden soll, wird die Alveole 57 mit einem Bearbeitungswerkzeug 58 vorbearbeitet, dadurch, dass in der Alveolenwand 7 den stufenartigen Querschnittsreduktionen 13 des Implantates entsprechende Schultern 13' erstellt werden (in Figur 22 rechts dargestellte Variante mit bearbeiteter Alveole 57'). Für diese Vorbearbeitung wird ein an den Wurzelbereich 11 angepasstes Bearbeitungswerkzeug 58 in die Alveole eingeführt. Die Querschnittsabmessungen des Bearbeitungswerkzeuges 58 sollen dabei leicht kleiner sein als die entsprechenden Abmessungen des Implantates. Wenn notwendig, können auch mehrere solche Bearbeitungswerkzeuge zur Anwendung kommen, wobei jedes Werkzeug verglichen mit dem vorgehend verwendeten Werkzeug etwas dicker ist.

Ebenfalls mit entsprechenden Werkzeugen wird Alveole gegebenenfalls vorbereitet, wenn nicht ein individuell an die Alveole angepasstes sondern ein geeignetes aber standardisiertes Implantat verwendet werden soll.

Die Bearbeitungswerkzeuge 58 können mit entsprechenden Schlägen in die Alveole eingebracht werden. Vorteilhafterweise werden sie aber mit mechanischen Schwingungen, vorteilhafterweise Ultraschall, angeregt und gleichzeitig in die Alveole geführt. Gegebenenfalls kann das Bearbeitungswerkzeug 58 mit einem leicht abrasiven Medium umspült werden, welches Medium durch eine Öffnung am distalen Ende des Werkzeuges zwischen Werkzeug und Alveolenwand gepresst wird und welches Medium auch dazu dient, abgetragenes Knochenmaterial wegzutransportieren.

In die ausgeräumte oder entsprechend bearbeitete Alveole (57 oder 57') wird das Implantat 10 eingesetzt. Dazu wird das Implantat mit mechanischen Schwingungen, insbesondere mit Ultraschall beaufschlagt, was vorteilhafterweise während dem Einsetzen des Implantates in die Alveole geschieht. Selbstverständlich ist es auch möglich, das Implantat zuerst mit einem Schlagwerkzeug in der Alveole einzusetzen und erst dann mit Ultraschall zu beaufschlagen.

Insbesondere, wenn das Implantat einen Kronenbereich 12 aufweist, wird für das Einsetzen vorteilhafterweise ein an diesen Kronenbereich angepasstes Übergangselement 52 verwendet. Wenn das Implantat nur einen Wurzelbereich mit einer im wesentlichen flachen proximalen Stirnfläche oder einen normierten Aufbau aufweist, kann zur Implantation ebenfalls eine Übergangselement 52 oder aber eine entsprechende Standardsonotrode verwendet werden. Durch die Anpassung von Länge und Geometrie der Sonotrode und gegebenenfalls des Übergangselementes kann die akustische Anregung des Implantates optimiert werden. Die Sonotrode oder das Übergangselement 52 kann durch geeignete Massnahmen wie Stoff- oder Formschluss oder auch durch die Anwendung von Vakuum die Kopplung zum Implantat unterstützend und das Handling verbessernd ausgerüstet sein (siehe auch Figuren 19 und 20 und entsprechende Beschreibungsteile) .

Wenn im Wurzelbereich des Dentalimplantates nur die mechanisch relevanten Teile der entsprechenden natürlichen Zahnwurzel nachgebildet sind, diese aber ganz extrahiert wurde, sind vor der Implantation durch das Implantat nicht zu besetzende Alveolenteile vorteilhafterweise zu füllen mit einem Knochenersatzmaterial, z.B. mit Calziumphosphat-Granulat, wie es für Augmentationen verwendet wird.

Vorteilhafterweise wird das Implantat so schnell wie möglich, vorteilhafterweise unmittelbar nach der Extraktion des zu ersetzenden Zahnes implantiert.

Es ist selbstverständlich auch möglich, für die Implantation des erfindungsgemässen Implantates in einer Stelle eines Kieferknochens, die keine oder eine bereits verwachsene Alveole aufweist, eine Kavität zu erstellen und diese in der oben genannten Weise für die Implantation vorzubereiten. Die Form einer solchen Kavität und des entsprechenden Implantates kann dabei an Knochenstrukturen angepasst werden, die wie eine Alveole mittels Computertomogramm vermessen werden kann.

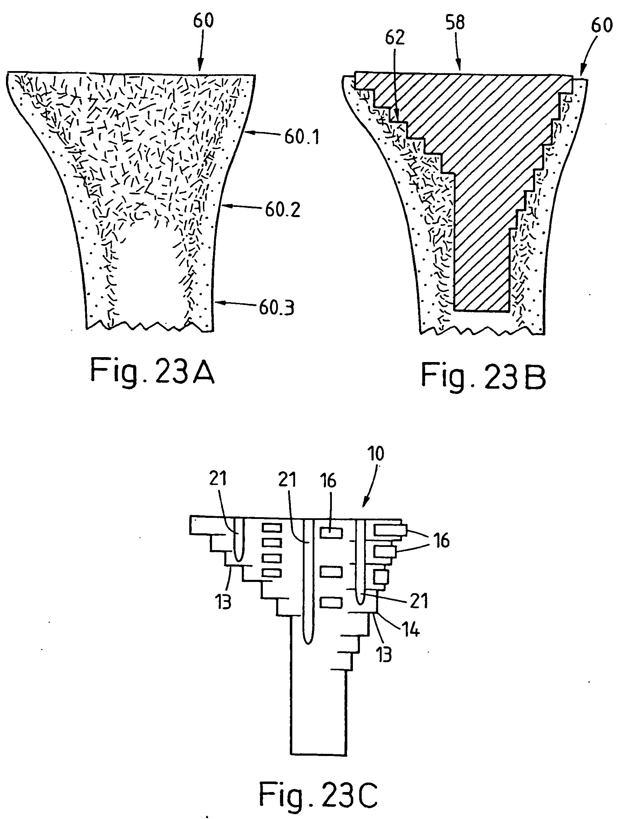

**Figuren 23A bis 23C** illustrieren die Implantation eines Gelenkimplantates gemäss Erfindung. Figur 23A zeigt einen Schnitt durch den Knochen 60 mit dem epiphysären Bereich 60.1, dem metaphysären Bereich 60.2 und dem diaphysären Bereich 60.3, in welchem Knochen das Gelenkimplantat, das ein eigens für den Knochen hergestelltes Individualimplantat oder ein geeignetes, standardisiertes Implantat sein kann, zu implantieren ist. Figur 23B zeigt das Bearbeitungsinstrument 58 (ebenfalls im Schnitt), dessen Form im wesentlichen mit der Implantatsform übereinstimmt und mit dessen Hilfe im Knochen 60 die Kavität 62 erstellt oder fertiggestellt wird. Figur 23C zeigt das in der Kavität 62 zu implantierende Gelenkimplantat 10 als Seitenansicht. Dieses hat die Form eines unregelmässigen Konus und weist stufenartige Querschnittsreduktionen 13 mit Schneidekanten 14, zwischen diesen angeordnete Oberflächenbereiche 16 aus einem thermoplastischen Material und axial verlaufende, furchende oder schneidende Strukturen 21 (Rippen) auf.

Ausgehend von der mit Hilfe von CT oder MRI aufgenommenen Knochengeometrie wird das Gelenkimplantat 10 und das Bearbeitungswerkzeug 58 ausgewählt oder gefertigt, in im wesentlichen derselben Art und Weise, wie im Zusammenhang mit der Figur 21 für das Dentalimplantat beschrieben ist. Dabei wird das Implantat 10 und die Kavität 62 derart geplant, dass die Verankerung mit Hilfe der Schneidekanten 14 und der Rippen 21 im epi- und metaphysären Bereich liegt. Die Oberflächenbereiche 16 aus dem thermoplastischen Material sind an Stellen angeordnet, die erhöhten Zug- und Schubspannungen ausgesetzt sind. Dadurch werden gezielt osseointegrationsmindernde Verschiebungen oder Dehnungen in der Grenzfläche zwischen Implantat und Knochen auf ein unkritisches Mass reduziert. Bei der Erstellung der Kavität kann die erste Öffnung mit Standard-Instrumenten erstellt werden. Mindestens für den letzten Räumungsschritt wird das formmässig an das Implantat angepasste Bearbeitungswerkzeug 58 verwendet, um damit die Form der Kavität 62 ausreichend nahe an die Form des Implantates 10 anzupassen.

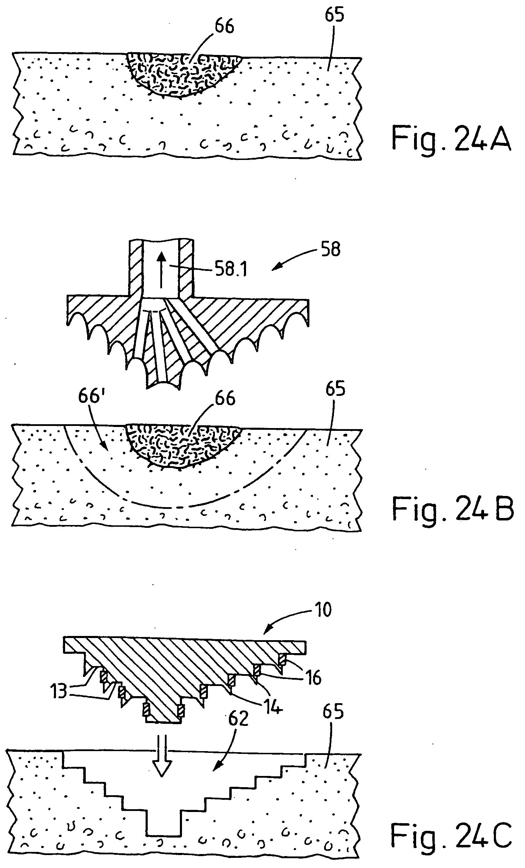

**Figuren 24A bis 24C** illustrieren die Reparatur einer durch Entfernung eines Knochentumors entstandenen Knochendefektes mit einem defektüberbrückenden, erfindungsgemässen Implantat 10. Figur 24A zeigt den geschnittenen Knochen 65 mit dem Tumor 66. Figur 24B zeigt im Schnitt den zu entfernenden Knochenbereich 66' (Excission) und das mindestens für die Fertigstellung der Kavität 62 eingesetzte Bearbeitungswerkzeug 58. Figur 24C zeigt im Schnitt die fertig gestellte Kavität 62 und das in der Kavität zu implantierende Implantat 10, das wiederum beispielsweise die Form eines unregelmässigen Konus hat und stufenartige Querschnittsreduktionen 13 mit Schneidekante 14 und Oberflächenbereiche 16 aus einem thermoplastischen Material aufweist.

Der Knochentumor 66 wird vorgängig mittels Röntgenaufnahmen, CT oder MRI geometrisch eingegrenzt. Auf Basis der Vermessungsdaten wird vom Chirurgen die Grösse der Excission bestimmt. Das Implantat 10 und das Bearbeitungswerkzeug 58 werden entsprechend der Excissionsgeometrie ausgewählt oder eigens dafür hergestellt.

Das Bearbeitungswerkzeug 58 weist ferner Absaugkanäle 58.1 auf, die im Bereiche der schneidenförmig ausgebildeten Kanten der Querschnittsreduktionen in die Werkzeugoberfläche münden. Durch diese Absaugkanäle 58.1 werden Knochenmaterial, Knochenmark und Tumorzellen aus der Kavität abgesaugt, wodurch das mit dem Werkzeug 58 räumbare Volumen erhöht und der Aufbau von lokal hohen Drücken, die zu Fettembolien führen könnten, vermieden wird. Durch das Absaugen der Tumorzellen wird auch verhindert, dass diese ins gesunde Gewebe transferiert werden, womit das Risiko, metastasierende Zellen zurückzulassen, erheblich reduziert wird.

Die vorgängig beschriebenen Figuren und die entsprechenden Beschreibungsteile beziehen sich in den meisten Fällen auf spezifische Implantate (Dentalimplantat, Gelenkimplantat, Individualimplantat, Standardimplantat etc.) und auf spezifische Merkmale dieser Implantate. Selbstverständlich ist es möglich, die beschriebenen Merkmale an anderen Implantaten anzuwenden und anders zu kombinieren, als dies in der vorliegenden Beschreibung der Fall ist. Der Gegenstand der Erfindung ist durch die Ansprüche definiert.

## Patentansprüche

1. Knochenimplantat (10), das für die Implantation in einer Implantationsrichtung parallel zu einer Implantatsachse (I) in einer von einer Kavitätswand (K) aus Knochengewebe (3) umgebenen Kavität geeignet ist, wobei das Implantat einen zu implantierenden Bereich aufweist,
wobei der zu implantierende Bereich ferner Schneidekanten (14) aufweist,
o die nicht in einer mit der Implantatsachse (I) gemeinsamen Ebene liegen,
o die gegen einen distalen Endbereich des Implantats gerichtet sind,
o und die sich mindestens teilweise um den Umfang des Implantats erstrecken,
wobei die Schneidekanten (14) als Aussenkanten stufenartiger Reduktionen des Querschnitts zum distalen Endbereich hin ausgebildet sind, so dass sich das Implantat in distaler Richtung verjüngt, oder der zu implantierende Bereich im Wesentlichen zylindrisch ist und die Schneidekanten (14) aus der Zylinder-form ragen und in Implantationsrichtung abnehmende Abstände von der Implantatsachse (I) aufweisen;
wobei das Implantat in einem zu implantierenden Bereich gemäss einer ersten Alternative Oberflächenbereiche (16) aus einem durch mechanische Schwingungen verflüssigbaren Material (M) aufweist oder solche Oberflächenbereiche (16) gemäss einer zweiten Alternative durch Auspressen des verflüssigbaren Materials aus einem Hohlraum (26) durch Öffnungen (27) erstellbar sind,
und wobei gemäss der ersten Alternative die Schneidekanten (14) ausserhalb der Oberflächenbereiche (16) aus dem verflüssigbaren Material sind und gemäss der zweiten Alternative die Öffnungen (27) den Hohlraum mit Oberflächenbereichen ohne Schneidekanten verbinden.

2. Knochenimplantat nach Anspruch 1, **dadurch gekennzeichnet, dass** die Schneidekanten (14) einen Keilwinkel (β) von weniger als 90° aufweisen.

3. Knochenimplantat nach Anspruch 1 oder 2, **dadurch gekennzeichnet, dass** die Schneidekanten (14) leicht ausladend gestaltet sind.

4. Knochenimplantat nach einem der Ansprüche 1 bis 3, **dadurch gekennzeichnet, dass** die Schneidekanten (14) zu einem Spanraum (23) hinterschnitten sind.

5. Knochenimplantat nach einem der Ansprüche 1 bis 4, **dadurch gekennzeichnet, dass** das verflüssigbare Material (M) in Vertiefungen (40) angeordnet ist und die Oberflächenbereiche (16) aus dem verflüssigbaren Material (M) über Oberflächenbereiche (17) neben den Vertiefungen (40) vorstehen.

6. Knochenimplantat nach einem der Ansprüche 1 bis 4, **dadurch gekennzeichnet, dass** die Öffnungen (27) in Vertiefungen (40) münden.

7. Knochenimplantat nach einem der Ansprüche 5 oder 6, **dadurch gekennzeichnet, dass** die Vertiefungen (40) Nuten sind, die sich axial oder spiralig über den zu implantierenden Bereich erstrecken.

8. Knochenimplantat nach einem der Ansprüche 1 bis 7, **dadurch gekennzeichnet, dass** zwischen den Oberflächenbereichen (16) aus dem verflüssigbaren Material osseointegrative Oberflächenbereiche (17) angeordnet sind.

9. Knochenimplantat nach einem der Ansprüche 1 bis 8, **dadurch gekennzeichnet, dass** am zu implantierenden Bereich ferner sich axial erstreckende, furchende oder schneidende Strukturen (21) vorgesehen sind.

10. Knochenimplantat nach einem der Ansprüche 1 bis 9, **dadurch gekennzeichnet, dass** die teilweise um den Implantatsumfang verlaufenden Schneidkanten (14) Unterkanten von schuppenartigen Strukturen bilden.

11. Knochenimplantat nach einem der Ansprüche 1 bis 10, **dadurch gekennzeichnet, dass** der proximale Endbereich einen Kragen (31) mit einer schneidenartig ausgebildeten Unterkante aufweist.

12. Knochenimplantat nach einem der Ansprüche 1 bis 11, **dadurch gekennzeichnet, dass** der proximale Endbereich einen Ring (32) aus einem thermoplastischen Material aufweist.

13. Knochenimplantat nach einem der vorangehenden Ansprüche, **gekennzeichnet durch** weitere stufenartigen Querschnittsreduktionen (13) in der Form nicht schneidender Kanten mit einem Keilwinkel (β) von 90° oder mehr.

14. Knochenimplantat nach einem der vorangehenden Ansprüche, **dadurch gekennzeichnet, dass** die aus der Zylinderform ragenden Schneidekanten (14) um einen Teil des Implantatumfangs laufen und in Serien in axialer Richtung aufeinander ausgerichtet sind.

15. Knochenimplantat nach Anspruch 14, **dadurch gekennzeichnet, dass** es mindestens zwei einander gegenüberliegende Serien von Schneidekanten (14, 14', 14") aufweist und dass die Oberflächenbereiche (16) aus dem verflüssigbaren Material (M) oder Mündungen der Öffnungen (27) am Implantatsumfang zwischen den Serien angeordnet sind.

16. Knochenimplantat nach einem der vorangehenden Ansprüche, **dadurch gekennzeichnet, dass** es einen inneren Hohlraum (26) aufweist und einen in eine proximale Öffnung des Hohlraums (26) einschiebbaren Kolben (42).

17. Knochenimplantat nach Anspruch 16, **dadurch gekennzeichnet, dass** an einem proximalen Ende (43) des Kolbens (42) und/oder rund um die proximale Öffnung des Hohlraumes (26) Mittel für eine dichtende Verbindung von Kolben (42) und Implantat vorgesehen sind.

18. Knochenimplantat nach einem der vorangehenden Ansprüche, **dadurch gekennzeichnet, dass** es an einem proximalen Endbereich ein Übergangselement (52) trägt.

19. Knochenimplantat nach Anspruch 18, **dadurch gekennzeichnet, dass** das Übergangselement (52) über eine Spielpassung an das Implantat anschliesst und/oder für eine Verbindung mit Spielpassung an eine Sonotrode (53) ausgerüstet ist.

20. Knochenimplantat nach einem der vorangehenden Ansprüche, **dadurch gekennzeichnet, dass** es ein Dentalimplantat (10) ist.

21. Knochenimplantat nach Anspruch 20, **dadurch gekennzeichnet, dass** es zusätzlich zum Wurzelbereich (11) einen Kronenbereich (12), einen Abutmentteil (30) oder Mittel (20) zum Befestigen eines Abutments, einer Krone (19), einer Brücke oder einer Zahnprothese aufweist.

22. Knochenimplantat nach einem der Ansprüche 1 bis 19, **dadurch gekennzeichnet, dass** es der Schaft einer Gelenkprothese ist.

23. Knochenimplantat nach einem der Ansprüche 1 bis 19, **dadurch gekennzeichnet, dass** es für die Überbrückung eines Knochendefekts ausgelegt ist.

24. Knochenimplantat nach einem der vorangehenden Ansprüche, **dadurch gekennzeichnet, dass** im Hohlraum (26) Energierichtungsgeber (28) vorgesehen sind.

25. Implantations-Set bestehend aus einem Knochenimplantat nach einem der Ansprüche 1 bis 24 und mindestens einem Bearbeitungswerkzeug (58), das form-mässig an den zu implantierenden Bereich des Implantats angepasst ist, und/oder einem Übergangselement (52), das formmässig an einen proximalen Endbereich des Implantats angepasst ist.

26. Verfahren zur Herstellung eines Knochenimplantates nach einem der Ansprüche 1 bis 24, welches Knochenimplantat als Ersatz eines Knochenteils oder eines Zahns in einer vorgegebenen oder zu erstellenden Kavität mit einer knöchernen Kavitätswand (K) implantiert wird, wobei das Verfahren einen Vermessungsschritt aufweist, in dem der zu ersetzende Knochenteil oder Zahn (1) und/oder die vorgegebene Kavität oder eine vorgegebene Knochenstruktur im Bereiche der zu erstellenden Kavität vermessen wird zur Erstellung von Messdaten, durch die der Knochenteil, der Zahn (1), die Kavität oder die Knochenstruktur abbildbar sind, einen Messdaten-Verarbeitungsschritt, in dem die Messdaten bearbeitet werden, und einen Herstellungsschritt, in dem anhand der bearbeiteten Messdaten das Knochenimplantat (10) hergestellt wird, **dadurch gekennzeichnet, dass** im Messdaten-Verarbeitungsschritt der zu implantierende Bereich mit Schneidekanten (14) versehen wird, die derart dimensioniert sind, dass sie nach der Implantation mindestens teilweise in der Kavitätswand eingeschnitten sind, und dass der zu implantierende Bereich mit Strukturen zur Aufnahme von Teilen aus dem verflüssigbaren Material (M) ausgerüstet ist.

27. Verfahren nach Anspruch 26, **dadurch gekennzeichnet, dass** im Vermessungsschritt ein dreidimensionales Bild oder ein oder mehrere zweidimensionale Bilder aufgenommen werden.

28. Verfahren nach Anspruch 26 oder 27, **dadurch gekennzeichnet, dass** im Messdaten-Verarbeitungsschritt im zu implantierenden Bereich ein innerer Hohlraum (26) und Öffnungen (27), die die Oberfläche des zu implantierenden Bereichs mit dem inneren Hohlraum (26) verbinden, konstruiert werden.

29. Verfahren nach einem der Ansprüche 26 bis 28, **dadurch gekennzeichnet, dass** im Messdaten-Verarbeitungsschritt am zu implantierenden Bereich axial verlaufende, furchende oder schneidende Strukturen (21) konstruiert werden, die derart dimensioniert sind, dass sie nach der Implantation mindestens teilweise in die Kavitätswand reichen.

30. Verfahren nach einem der Ansprüche 26 bis 29, **dadurch gekennzeichnet, dass** im Messdaten-Verarbeitungsschritt zusätzlich Daten für die Herstellung eines an den zu implantierenden Bereich des Implantates angepassten Bearbeitungswerkzeuges (58) erstellt werden.

31. Verfahren nach einem der Ansprüche 26 bis 30, **dadurch gekennzeichnet, dass** im Messdaten-Verarbeitungsschritt zusätzlich Daten für die Herstellung eines an den proximalen Endbereich des Implantates angepassten Übergangselements (52) erstellt werden.

32. Verfahren nach einem der Ansprüche 26 bis 31, **dadurch gekennzeichnet, dass** im Messdaten-Verarbeitungsschritt osseointegrativ wirkende Strukturen konstruiert werden.

33. Verfahren nach einem der Ansprüche 26 bis 32, **dadurch gekennzeichnet, dass** das Knochenimplantat ein Dentalimplantat (10) ist und dass der zu ersetzende Zahn (1) und/oder die Alveole (57) vor der Extraktion des zu ersetzenden Zahnes (1) vermessen werden.

## Claims

1. A bone implant (10) which is suitable for the implantation in a cavity surrounded by a cavity wall (K) of bone tissue (3), in an implantation direction parallel to an implant axis (I), wherein the implant comprises a region to be implanted,
wherein the region to be implanted further comprises cutting edges (14),
∘ which do not lie in a common plane with the implant axis (I),
∘ which are directed towards a distal end region of the implant,
∘ and which extend at least partly around the periphery of the implant,
wherein the cutting edges (14) are designed as outer edges of step-like reductions of the cross section towards the distal end region, so that the implant tapers in a distal direction, or the region to be implanted is essentially cylindrical and the cutting edges (14) project out of the cylinder shape and have distances to the implant axis (I) which decrease in the implantation direction,
wherein the implant in a region to be implanted, according to a first alternative comprises surface regions (16) of a material (M) liquefiable by way of mechanical oscillations, or such surface regions (16) according to a second alternative can be created by way of pressing the liquefiable material out of a cavity (26) through openings (27),
and wherein according to the first alternative, the cutting edges (14) are arranged outside the surface regions (16) of the liquefiable material and according to the second alternative the openings (27) connect the cavity to surface regions without cutting edges.

2. The bone implant according to claim 1, **characterised in that** the cutting edges (14) have a wedge angle (β) of less than 90°.

3. The bone implant according to claim 1 or 2, **characterised in that** the cutting edges (14) are designed in a slightly protruding manner.

4. The bone implant according to one of the claims 1 to 3, **characterised in that** the cutting edges are undercut into a chip space (23).

5. The bone implant according to one of the claims 1 to 4, **characterised in that** the liquefiable material (M) is arranged in recesses (40), and the surface regions (16) of the liquefiable material (M) project beyond surface regions (17) next to the recesses (40).

6. The bone implant according to one of the claims 1 to 4, **characterised in that** the openings (27) run out into recesses (40).

7. The bone implant according to claim 5 or 6, **characterised in that** the recesses (40) are grooves which extend axially or spirally over the region to be implanted.

8. The bone implant according to one of the claims 1 to 7, **characterised in that** osseo-integrative surface regions (17) are arranged between the surface regions (16) of the liquefiable material.

9. The bone implant according to one of the claims 1 to 8, **characterised in that** moreover axially extending, furrowing or cutting structures (21) are provided on the region to be implanted.

10. The bone implant according to one of the claims 1 to 9, **characterised in that** the cutting edges (14) which run partly around the implant periphery form lower edges of scale-like structures.

11. The bone implant according to one of the claims 1 to 10, **characterised in that** the proximal end region comprises a collar (31) with a lower edge designed in a cutting-edge-like manner.

12. The bone implant according to one of the claims 1 to 11, **characterised in that** the proximal end region comprises a ring (32) of a thermoplastic material.

13. The bone implant according to one of the preceding claims, **characterised by** further step-like cross-sectional reductions (13) in the form of non-cutting edges with a wedge angle (ß) of 90° or more.

14. The bone implant according to one of the preceding claims, **characterised in that** the cutting edges (14) which project out of the cylinder shape run around a part of the implant periphery, and in series are aligned to one another in the axial direction.

15. The bone implant according to claim 14, **characterised in that** it comprises at least two oppositely lying series of cutting edges (14, 14', 14") and that the surface regions (16) of the liquefiable material (M), or run-outs of the openings (27), are arranged between the series on the implant periphery.

16. The bone implant according to one of the preceding claims, **characterised in that** it comprises an inner cavity (26) and a piston (42) which can be inserted into a proximal opening of the cavity (26).

17. The bone implant according to claim 16, **characterised in that** means for a sealing connection of the piston (42) and the implant are provided at a proximal end (43) of the piston (42) and/or around the proximal opening of the cavity (26).

18. The bone implant according to one of the preceding claims, **characterised in that** it carries an intermediate element (52) at a proximal end region.

19. The bone implant according to claim 18, **characterised in that** the intermediate element (52) connects to the implant via a clearance fit and/or is designed for a connection comprising a clearance fit to a sonotrode (53).

20. The bone implant according to one of the preceding claims, **characterised in that** it is a dental implant (10).

21. The bone implant according to claim 20, **characterised in that** additionally to the root region (11) it comprises a crown region (12), an abutment part (30) or means (20) for fastening an abutment, a crown (19), a bridge or a dental prosthesis.

22. The bone implant according to one of the claims 1 to 19, **characterised in that** it is the shank of a joint prosthesis.

23. The bone implant according to one of the claims 1 to 19, **characterised in that** it is designed for bridging a bone defect.

24. The bone implant according to one of the preceding claims, **characterised in that** energy directors (28) are provided in the cavity (26).

25. An implantation set comprising a bone implant according to one of the claims 1 to 24 and at least one processing tool (58) which with regard to shape is adapted to the region to be implanted of the implant, and/or an intermediate element (52) which with regard to shape is adapted to a proximal end region of the implant.

26. A method for manufacturing a bone implant according to one of the claims 1 to 24, said bone implant as a replacement of a bone part or of a tooth being implanted in a given cavity or one to be created, with a bony cavity wall (K), wherein the method comprises a measuring step, in which the bone part or tooth (1) which is to be replaced, and/or the given cavity or a given bone structure in the region of the cavity to be created is measured for creating measurement data, by way of which the bone part, the tooth (1), the cavity or the bone structure can be modelled, a measurement data processing step, in which the measurement data is processed, and a manufacturing step, in which the bone implant (10) is manufactured by way of the processed measurement data, **characterised in that** the region to be implanted is provided with cutting edges (14) in the measurement data processing step, and these cutting edges are dimensioned in a manner such that after the implantation they are at least partly cut into the cavity wall, and that the region to be implanted is provided with structures for receiving parts of the liquefiable material (M).

27. The method according to claim 26, **characterised in that** a three-dimensional picture or one or more two-dimensional pictures are taken in the measuring step.

28. The method according to claim 26 or 27, **characterised in that** an inner cavity (26) and openings (27) which connect the surface of the region to be implanted to the inner cavity (26) are designed in the region to be implanted, in the measurement data processing step.

29. The method according to one of the claims 26 to 28, **characterised in that** axially running, furrowing or cutting structures (21) are designed on the region to be implanted, in the measurement data processing step, wherein these structures (21) are dimensioned in a manner such that they at least partly reach into the cavity wall after implantation.

30. The method according to one of the claims 26 to 29, **characterised in that** additionally, data for the manufacture of a processing tool (58) which is adapted to the region of the implant which is to be implanted, are created in the measurement data processing step.

31. The method according to one of the claims 26 to 30, **characterised in that** data for the manufacture of an intermediate element (52) which is adapted to the proximal end region of the implant are additionally created in the measurement data processing step.

32. The method according to one of the claims 26 to 31, **characterised in that** osseo-integratively acting structures are designed in the measurement data processing step.

33. The method according to one of the claims 26 to 32, **characterised in that** the bone implant is a dental implant (10) and that the tooth (1) which is to be replaced and/or the alveole (57) are measured before the extraction of the tooth (1) to be replaced.

## Revendications

1. Implant (10) pour os convenant pour être implanté dans une direction d'implantation parallèle à un axe (I) de l'implant dans une cavité entourée par une paroi (K) de cavité en tissu osseux (3),
l'implant présentant une partie à implanter,
la partie à implanter présentant en outre des arêtes de coupe (14)
∘ qui ne sont pas situées dans un plan commun avec l'axe (I) de l'implant,
∘ qui sont orientées vers une partie d'extrémité distale de l'implant et
∘ qui s'étendant au moins en partie sur la périphérie de l'implant,
les arêtes de coupe (14) étant configurées comme arêtes extérieures de réductions en gradins de la section transversale en direction de la partie d'extrémité distale de telle sorte que l'implant se rétrécit dans la direction distale ou
la partie à implanter étant essentiellement cylindrique et les arêtes de coupes (14) débordant hors de la forme du cylindre et présentant dans la direction d'implantation des distances décroissantes par rapport à l'axe (I) de l'implant,
tandis que dans une première variante, l'implant présente dans une partie à implanter des parties de surface (16) en un matériau (M) apte à se liquéfier sous l'action de vibrations mécaniques ou que dans une deuxième variante, ces parties de surface (16) peuvent être formées par expulsion du matériau liquéfiable hors d'une cavité (26) par des ouvertures (27),
tandis que dans la première variante, les arêtes de coupe (14) sont situées à l'extérieur des parties de surface (16) en matériau liquéfiable et que dans la deuxième variante, les ouvertures (27) relient la cavité aux parties de surface non pourvues d'arêtes de coupe.

2. Implant pour os selon la revendication 1, **caractérisé en ce que** les arêtes de coupe (14) présentent un angle de biseau (β) inférieur à 90°.

3. Implant pour os selon les revendications 1 ou 2, **caractérisé en ce que** les arêtes de coupe (14) sont en léger délestage.

4. Implant pour os selon l'une des revendications 1 à 3, **caractérisé en ce que** les arêtes de coupe (14) présentent une contre-dépouille en direction d'un espace de puce (23).

5. Implant pour os selon l'une des revendications 1 à 4, **caractérisé en ce que** le matériau liquéfiable (M) est disposé dans des creux (40) et **en ce que** les parties de surface (16) en matériau liquéfiable (M) débordent au-delà des parties de surface (17) situées à côté des creux (40).

6. Implant pour os selon l'une des revendications 1 à 4, **caractérisé en ce que** les ouvertures (27) débouchent dans des creux (40).

7. Implant pour os selon l'une des revendications 5 ou 6, **caractérisé en ce que** les creux (40) sont des rainures qui s'étendent axialement ou en spirale sur la partie à implanter.

8. Implant pour os selon l'une des revendications 1 à 7, **caractérisé en ce que** des parties de surface (17) d'ostéointégration sont disposées entre les parties de surface (16) en matériau liquéfiable.

9. Implant pour os selon l'une des revendications 1 à 8, **caractérisé en ce que** des structures (21) de formation de sillon ou de découpe s'étendant axialement sont en outre prévues sur la partie à implanter.

10. Implant pour os selon l'une des revendications 1 à 9, **caractérisé en ce que** les arêtes de coupe (14) qui s'étendent en partie autour de la périphérie de l'implant forment les arêtes inférieures de structures en écailles.

11. Implant pour os selon l'une des revendications 1 à 10, **caractérisé en ce que** la partie d'extrémité proximale présente un collet (31) doté d'un bord inférieur en forme de lame.

12. Implant pour os selon l'une des revendications 1 à 11, **caractérisé en ce que** la partie d'extrémité proximale présente un anneau (32) en matériau thermoplastique.

13. Implant pour os selon l'une des revendications précédentes, **caractérisé par** d'autres réductions en gradins (13) de la section transversale présentant la forme d'arêtes non tranchantes dont l'angle de biseau (β) est de 90° ou davantage.

14. Implant pour os selon l'une des revendications précédentes, **caractérisé en ce que** les arêtes de coupe (40) débordant hors de la forme du cylindre s'étendent sur une partie de la périphérie de l'implant et sont alignées les unes sur les autres en série dans la direction axiale.

15. Implant pour os selon la revendication 14, **caractérisé en ce qu'**il présente au moins deux séries mutuellement opposées d'arêtes de coupe (14, 14', 14") et **en ce que** les parties de surface (16) en matériau liquéfiable (M) ou les embouchures des ouvertures (27) sont disposées à la périphérie de l'implant entre les séries.

16. Implant pour os selon l'une des revendications précédentes, **caractérisé en ce qu'**il présente une cavité intérieure (26) et un piston (42) qui peut être inséré dans une ouverture proximale de la cavité (26).

17. Implant pour os selon la revendication 16, **caractérisé en ce qu'**à une extrémité proximale (43) du piston (42) et/ou autour de l'ouverture proximale de la cavité (26), des moyens de liaison hermétique entre le piston (42) et l'implant sont prévus.

18. Implant pour os selon l'une des revendications précédentes, **caractérisé en ce qu'**il porte une pièce intermédiaire (52) sur une partie d'extrémité proximale.

19. Implant pour os selon la revendication 18, **caractérisé en ce que** la pièce intermédiaire (52) se raccorde à l'implant par l'intermédiaire d'un ajustement avec jeu et/ou est équipé pour être relié avec un ajustement avec jeu à une sonotrode (53).

20. Implant pour os selon l'une des revendications précédentes, **caractérisé en ce qu'**il est un implant dentaire (10).

21. Implant pour os selon la revendication 20, **caractérisé en ce qu'**il présente en plus de la partie de racine (11) une partie de couronne (12), une partie de butée (30) ou des moyens (20) de fixation d'une butée, d'une couronne (19), d'un pontage ou d'une prothèse dentaire.

22. Implant pour os selon l'une des revendications 1 à 19, **caractérisé en ce qu'**il forme la tige d'une prothèse d'articulation.

23. Implant pour os selon l'une des revendications 1 à 19, **caractérisé en ce qu'**il est conçu pour ponter un défaut osseux.

24. Implant pour os selon l'une des revendications précédentes, **caractérisé en ce que** des orienteurs (28) d'énergie sont prévus dans la cavité (26).

25. Kit d'implantation comprenant un implant pour os selon l'une des revendications 1 à 24 et d'au moins un outil de traitement (58) dont la forme est adaptée à celle de la partie à implanter de l'implant et/ou une pièce intermédiaire (52) dont la forme est adaptée à une partie d'extrémité proximale de l'implant.

26. Procédé de fabrication d'un implant pour os selon l'une des revendications 1 à 24, l'implant pour os étant implanté pour remplacer une partie d'os ou une dent dans une cavité prédéterminée ou à réaliser dotée d'une paroi osseuse (K) de cavité, le procédé présentant
une étape de mesure dans laquelle la partie d'os ou la dent (1) à remplacer et/ou la cavité prédéterminée ou une structure osseuse prédéterminée sont mesurées au niveau de la cavité à former pour établir des données de mesure par lesquelles la partie d'os, la dent (1), la cavité ou la structure osseuse peuvent être représentées,
une étape de traitement des données de mesure dans laquelle les données de mesure sont traitées et
une étape de fabrication dans laquelle, l'implant (10) pour os est fabriqué à l'aide des données de mesure traitées,
**caractérisé en ce que**
au cours de l'étape de traitement des données de mesure, la partie à implanter est dotée d'arêtes de coupe (14) dimensionnées de telle sorte qu'après l'implantation, elles pénètrent au moins en partie dans la paroi de la cavité et **en ce que** la partie à implanter est équipée de structures de reprise de parties en matériau liquéfiable (M).

27. Procédé selon la revendication 26, **caractérisé en ce que** dans l'étape de mesure, une image en trois dimensions ou une ou plusieurs images en deux dimensions sont enregistrées.

28. Procédé selon les revendications 26 ou 27, **caractérisé en ce que** dans l'étape de traitement des données de mesure, une cavité intérieure (26) et des ouvertures (27) qui relient la surface de la partie à implanter à la cavité intérieure (26) sont construites dans la partie à implanter.

29. Procédé selon l'une des revendications 26 à 28, **caractérisé en ce que** dans l'étape de traitement des données de mesure, des structures (21) formant des sillons ou de coupe, s'étendant axialement sur la partie à implanter, sont construites et dimensionnées de manière à, après l'implantation, pénétrer au moins en partie dans la paroi de la cavité.

30. Procédé selon l'une des revendications 26 à 29, **caractérisé en ce que** dans l'étape de traitement des données de mesure, des données supplémentaires de fabrication d'un outil de traitement (58) adapté à la partie à implanter de l'implant sont formées.

31. Procédé selon l'une des revendications 26 à 30, **caractérisé en ce que** dans l'étape de traitement des données de mesure, des données supplémentaires de fabrication d'une pièce intermédiaire (52) adapté à la partie d'extrémité proximale de l'implant sont formées.

32. Procédé selon l'une des revendications 26 à 31, **caractérisé en ce que** dans l'étape de traitement des données de mesure, des structures à action d'ostéointégration sont construites.

33. Procédé selon l'une des revendications 26 à 32, **caractérisé en ce que** l'implant pour os est un implant dentaire (10) et **en ce que** la dent (1) à remplacer et/ou l'alvéole (57) sont mesurées avant l'extraction de la dent (1) à remplacer.
